# EUROPEAN PATENT APPLICATION

(11) **EP 3 121 290 A1**
(43) Date of publication of application: **25.01.2017**
(21) Application number: 15764662.1
(22) Date of filing: 16.03.2015
(51) Int. Cl.: C12Q 1/68

(54) **PROGNOSIS METHOD OF AUTOIMMUNE DISEASES BY GENOTYPING GENETIC VARIATIONS OF THE VASOACTIVE INTESTINAL PEPTIDE**

(30) Priority: 17.03.2014 ES 201430364
(71) Applicant: Fundación Para La Investigación Biomédica Del Hospital De La Princesa, 28006 Madrid (ES); Universidad Complutense De Madrid, 28015 Madrid (ES)
(72) Inventor: GONZALEZ ALVARO, Isidoro, E-28006 Madrid (ES); ORTIZ GARCIA, Ana María, E-28006 Madrid (ES); LAMANA DOMINGUEZ, Amalia, E-28006 Madrid (ES); PEREZ GOMARIZ, Rosa, E-28006 Madrid (ES); MARTINEZ MORA, María del Carmen, E-28006 Madrid (ES); LECETA MARTINEZ, Javier, E-28006 Madrid (ES); JUARRANZ MORATILLA, Yasmina, E-28006 Madrid (ES)
(74) Representative: Temiño Ceniceros, Ignacio
(86) International application number: PCT/ES2015/070182
(87) International publication number: WO 2015/140375

(57) **Abstract**

The present invention relates to a prognosis method for determining the severity of autoimmune diseases, for example, rheumatoid arthritis, spondyloarthritis and systemic lupus erythematosus, characterized in that it comprises detecting certain genetic polymorphisms of the *VIP* gene including, among others, rs35643203, rs71575932 and/or rs7755568. The method of the invention is also useful for establishing a customized patient treatment. The invention also relates to the use of the genetic polymorphisms of the *VIP* gene described in the invention as biomarkers of said diseases, as well as to a kit comprising primers or probes for detecting said polymorphisms and to the use thereof for the same purpose.

SEQUENCE LISTING

<110>  Fundación de Investigación Biomédica del Hospital
       Universitario de la Princesa
       Universidad Complutense de Madrid

<120>  Prognosis method of autoimmune diseases by genotyping genetic
variations of the vasoactive intestinal peptide

<130>  ES1595.52

<160>  61

<170>  PatentIn version 3.5

<210>  1
<211>  28
<212>  PRT
<213>  Homo sapiens

<400>  1

His Ser Asp Ala Val Phe Thr Asp Asn Tyr Thr Arg Leu Arg Lys Gln
1               5                   10                  15

Met Ala Val Lys Lys Tyr Leu Asn Ser Ile Leu Asn
            20                  25

<210>  2
<211>  24
<212>  DNA
<213>  Artificial sequence

<220>
<223>  PRIMER FORWARD-AMPLICON 1

<400>  2
tcagagctgt caactgggaa acaa                                              24

<210>  3
<211>  20
<212>  DNA
<213>  Artificial sequence

<220>
<223>  PRIMER REVERSE - AMPLICON  1

<400>  3
tggcacaatt cccaatgcac                                                   20

<210>  4
<211>  26
<212>  DNA
<213>  Artificial sequence

<220>
<223>  PRIMER FORWARD - AMPLICON 2

<400>  4

tggtttgagt atgtgtgtgt gttggg                                                26

<210>  5
<211>  20
<212>  DNA
<213>  Artificial sequence

<220>
<223>  PRIMER REVERSE - AMPLICON 2

<400>  5
ggccatggga tgaatagggc                                                      20

<210>  6
<211>  20
<212>  DNA
<213>  Artificial sequence

<220>
<223>  PRIMER FORWARD - AMPLICON 3

<400>  6
tgaaggaatc ccaatggccc                                                      20

<210>  7
<211>  21
<212>  DNA
<213>  Artificial sequence

<220>
<223>  PRIMER REVERSE - AMPLICON 3

<400>  7
gttccagagg caggagggaa a                                                    21

<210>  8
<211>  20
<212>  DNA
<213>  Artificial sequence

<220>
<223>  PRIMER FORWARD - AMPLICON 4

<400>  8
gagcacgact gggcgaggta                                                      20

<210>  9
<211>  25
<212>  DNA
<213>  Artificial sequence

<220>
<223>  PRIMER REVERSE - AMPLICON 4

<400>  9
catacgaaat aggaaaaaaa aagca                                                25

<210>  10
<211>  26
<212>  DNA
<213>  Artificial sequence

<220>
<223>  PRIMER FORWARD - AMPLICON 5

<400>  10
tgtaagatgc tttaccctgc ttgctc                                            26


<210>  11
<211>  29
<212>  DNA
<213>  Artificial sequence

<220>
<223>  PRIMER REVERSE - AMPLICON 5

<400>  11
acattacttt ttcctctttc aattgtaga                                         29


<210>  12
<211>  25
<212>  DNA
<213>  Artificial sequence

<220>
<223>  PRIMER FORWARD - AMPLICON 6

<400>  12
tgcttttttt ttcctatttc gtatg                                             25


<210>  13
<211>  24
<212>  DNA
<213>  Artificial sequence

<220>
<223>  PRIMER REVERSE - AMPLICON 6

<400>  13
cagtgacagg tgcttgaata aaaa                                              24


<210>  14
<211>  20
<212>  DNA
<213>  Artificial sequence

<220>
<223>  PRIMER FORWARD - AMPLICON 7

<400>  14
gccaaccatg tgcccagaca                                                   20


<210>  15
<211>  25
<212>  DNA

<213>  Artificial sequence

<220>
<223>  PRIMER REVERSE - AMPLICON 7

<400>  15
gaataaaaag aaagtgaaag cctcc                                          25


<210>  16
<211>  24
<212>  DNA
<213>  Artificial sequence

<220>
<223>  PRIMER FORWARD - AMPLICON 8

<400>  16
atgtgttgag tgagaggtgt ttgt                                           24


<210>  17
<211>  23
<212>  DNA
<213>  Artificial sequence

<220>
<223>  PRIMER REVERSE - AMPLICON 8

<400>  17
cccgtaaact gaaaggaaaa ttc                                            23


<210>  18
<211>  25
<212>  DNA
<213>  Artificial sequence

<220>
<223>  PRIMER FORWARD - AMPLICON 9

<400>  18
ggaggctttc actttctttt tattc                                          25


<210>  19
<211>  24
<212>  DNA
<213>  Artificial sequence

<220>
<223>  PRIMER REVERSE - AMPLICON 9

<400>  19
tatctttcag tgatttggga gttg                                           24


<210>  20
<211>  23
<212>  DNA
<213>  Artificial sequence

<220>

<223>  PRIMER FORWARD - AMPLICON 10

<400>  20
gaattttcct ttcagtttac ggg                                              23


<210>  21
<211>  26
<212>  DNA
<213>  Artificial sequence

<220>
<223>  PRIMER REVERSE - AMPLICON 10

<400>  21
cctgttccaa acatctctga agaaca                                           26


<210>  22
<211>  24
<212>  DNA
<213>  Artificial sequence

<220>
<223> PRIMER FORWARD - AMPLICON 11

<400>  22
caactcccaa atcactgaaa gata                                             24


<210>  23
<211>  26
<212>  DNA
<213>  Artificial sequence

<220>
<223>  PRIMER REVERSE - AMPLICON 11

<400>  23
ttctttggtg taatttacta tgccaa                                           26


<210>  24
<211>  26
<212>  DNA
<213>  Artificial sequence

<220>
<223>  PRIMER FORWARD - AMPLICON 12

<400>  24
tgttcttcag agatgtttgg aacagg                                           26


<210>  25
<211>  24
<212>  DNA
<213>  Artificial sequence

<220>
<223>  PRIMER REVERSE - AMPLICON 12

<400>  25

cttgatcagg ttcatttgct ccct                                              24

<210>  26
<211>  26
<212>  DNA
<213>  Artificial sequence

<220>
<223>  PRIMER FORWARD - AMPLICON 13

<400>  26
ttggcatagt aaattacacc aaagaa                                            26

<210>  27
<211>  24
<212>  DNA
<213>  Artificial sequence

<220>
<223>  PRIMER REVERSE - AMPLICON 13

<400>  27
caatgatgga agagttgcca agga                                              24

<210>  28
<211>  23
<212>  DNA
<213>  Artificial sequence

<220>
<223>  PRIMER FORWARD - AMPLICON 14

<400>  28
atggcttcat tcatcctgat tta                                               23

<210>  29
<211>  25
<212>  DNA
<213>  Artificial sequence

<220>
<223>  PRIMER REVERSE - AMPLICON 14

<400>  29
caggcatttc taggagcaca tttca                                             25

<210>  30
<211>  24
<212>  DNA
<213>  Artificial sequence

<220>
<223>  PRIMER FORWARD - AMPLICON 15

<400>  30
caatgatgga agagttgcca agga                                              24

<210>  31
<211>  25
<212>  DNA
<213>  Artificial sequence

<220>
<223>  PRIMER REVERSE - AMPLICON 15

<400>  31
gaaagttgac ccaagagttt actga                                             25


<210>  32
<211>  25
<212>  DNA
<213>  Artificial sequence

<220>
<223>  PRIMER FORWARD - AMPLICON 16

<400>  32
tgaaatgtgc tcctagaaat gcctg                                             25


<210>  33
<211>  26
<212>  DNA
<213>  Artificial sequence

<220>
<223>  PRIMER REVERSE - AMPLICON 16

<400>  33
tccatgggct tgatagatgt tattga                                            26


<210>  34
<211>  25
<212>  DNA
<213>  Artificial sequence

<220>
<223>  PRIMER FORWARD - AMPLICON 17

<400>  34
tcagtaaact cttgggtcaa ctttc                                             25


<210>  35
<211>  20
<212>  DNA
<213>  Artificial sequence

<220>
<223>  PRIMER REVERSE - AMPLICON 17

<400>  35
ttttggcatg gcctgggact                                                   20


<210>  36
<211>  26
<212>  DNA

<213>  Artificial sequence

<220>
<223>  PRIMER FORWARD - AMPLICON 18

<400>  36
tcaataacat ctatcaagcc catgga                                          26


<210>  37
<211>  26
<212>  DNA
<213>  Artificial sequence

<220>
<223>  PRIMER REVERSE - AMPLICON 18

<400>  37
tgacgtttga ctggtacagg gtcttc                                          26


<210>  38
<211>  24
<212>  DNA
<213>  Artificial sequence

<220>
<223>  PRIMER FORWARD - AMPLICON 19

<400>  38
cagacaagat gacctctttg ccca                                            24


<210>  39
<211>  27
<212>  DNA
<213>  Artificial sequence

<220>
<223>  PRIMER REVERSE - AMPLICON 19

<400>  39
tccctatact taattgagga gctaaca                                         27


<210>  40
<211>  26
<212>  DNA
<213>  Artificial sequence

<220>
<223>  PRIMER FORWARD - AMPLICON  20

<400>  40
gaagaccctg taccagtcaa acgtca                                          26


<210>  41
<211>  24
<212>  DNA
<213>  Artificial sequence

<220>

<223>  PRIMER REVERSE - AMPLICON  20

<400>  41
tgggcaaaga ggtcatcttg tctg                                              24


<210>  42
<211>  20
<212>  DNA
<213>  Artificial sequence

<220>
<223>  PRIMER FORWARD - AMPLICON  21

<400>  42
cggtggtgtg gccctagaca                                                   20


<210>  43
<211>  24
<212>  DNA
<213>  Artificial sequence

<220>
<223>  PRIMER REVERSE - AMPLICON  21

<400>  43
ctccaaaggt ctttttcatc attt                                              24


<210>  44
<211>  24
<212>  DNA
<213>  Artificial sequence

<220>
<223>  PRIMER FORWARD - AMPLICON  22

<400>  44
cagacaagat gacctctttg ccca                                              24


<210>  45
<211>  27
<212>  DNA
<213>  Artificial sequence

<220>
<223>  PRIMER REVERSE - AMPLICON  22

<400>  45
tccctatact taattgagga gctaaca                                           27


<210>  46
<211>  24
<212>  DNA
<213>  Artificial sequence

<220>
<223>  PRIMER FORWARD - AMPLICON  23

<400>  46

aaatgatgaa aaagaccttt ggag                                                24

<210>  47
<211>  29
<212>  DNA
<213>  Artificial sequence

<220>
<223>  PRIMER REVERSE - AMPLICON  23

<400>  47
caaacttaca ggtaaaacaa gctaaattt                                           29

<210>  48
<211>  27
<212>  DNA
<213>  Artificial sequence

<220>
<223>  PRIMER FORWARD - AMPLICON  24

<400>  48
tgttagctcc tcaattaagt ataggga                                             27

<210>  49
<211>  24
<212>  DNA
<213>  Artificial sequence

<220>
<223>  PRIMER REVERSE - AMPLICON  24

<400>  49
gggtaattag tggacaaaag cagt                                                24

<210>  50
<211>  23
<212>  DNA
<213>  Artificial sequence

<220>
<223>  PRIMER FORWARD - AMPLICON  25

<400>  50
agccagattc caatccctaa cta                                                 23

<210>  51
<211>  24
<212>  DNA
<213>  Artificial sequence

<220>
<223>  PRIMER REVERSE - AMPLICON  25

<400>  51
accatttaca aagcacagtg tgat                                                24

<210>  52
<211>  24
<212>  DNA
<213>  Artificial sequence

<220>
<223>  PRIMER FORWARD - AMPLICON  26

<400>  52
actgcttttg tccactaatt accc                                              24


<210>  53
<211>  23
<212>  DNA
<213>  Artificial sequence

<220>
<223>  PRIMER REVERSE - AMPLICON  26

<400>  53
tctctttgtc ccttgttaac agc                                               23


<210>  54
<211>  24
<212>  DNA
<213>  Artificial sequence

<220>
<223>  PRIMER FORWARD - AMPLICON  27

<400>  54
atcacactgt gctttgtaaa tggt                                              24


<210>  55
<211>  23
<212>  DNA
<213>  Artificial sequence

<220>
<223>  PRIMER REVERSE - AMPLICON  27

<400>  55
atacagcata tgaaattgca ggc                                               23


<210>  56
<211>  23
<212>  DNA
<213>  Artificial sequence

<220>
<223>  PRIMER FORWARD - AMPLICON  28

<400>  56
gctgttaaca agggacaaag aga                                               23


<210>  57
<211>  26
<212>  DNA

<213>  Artificial sequence

<220>
<223>  PRIMER REVERSE – AMPLICON  28

<400>  57
cagcattcca tctgcttaac attcaa                                          26


<210>  58
<211>  23
<212>  DNA
<213>  Artificial sequence

<220>
<223>  PRIMER FORWARD – AMPLICON  29

<400>  58
gcctgcaatt tcatatgctg tat                                             23


<210>  59
<211>  20
<212>  DNA
<213>  Artificial sequence

<220>
<223>  PRIMER REVERSE – AMPLICON  29

<400>  59
tgagggatga gctggaaggg                                                 20


<210>  60
<211>  18
<212>  DNA
<213>  Artificial sequence

<220>
<223>  5′ FORWARD PRIMER – M13 VIRUS
<400>  60
tgtaaaacga cggccagt                                                   18


<210>  61
<211>  18
<212>  DNA
<213>  Artificial sequence

<220>
<223>  5′REVERSE PRIMER – M13 VIRUS

<400>  61
caggaaacag ctatgacc                                                   18

## Description

The present invention relates to a prognosis method for the prognosis of autoimmune diseases which comprises detecting genetic polymorphisms of the vasoactive intestinal peptide (*VIP*) gene. The present invention can therefore be comprised in the field of biomedicine.

### PRIOR ART

Autoimmune diseases, such as rheumatoid arthritis (RA), psoriasis, inflammatory bowel disease, spondyloarthritis or systemic lupus erythematosus, share a series of etiopathogenic mechanisms leading to them being generally called "immune-mediated inflammatory diseases" (IMIDs) and also share their response to similar or identical immunomodulating or immunosuppressive treatments. RA is the most common autoimmune rheumatic disease.

RA causes chronic inflammation of the synovial membrane of diarthrodial joints, which leads to progressive damage, and as a result, causes disability and deterioration of the patient's quality of life. Furthermore, it is frequently accompanied by systemic manifestations and an increased number of comorbidities and even mortality compared with the general population. It is considered to have a multigenic etiology with significant interaction with environmental factors. Its prevalence ranges between 0.3 and 1%, the prevalence in Spain being, for example, 0.5%, which means that there are about 200,000 RA patients in said country. Given its ability to cause functional disability, RA is one of the main causes of permanent work disability in Spain. Therefore, it is a disease with a high social and economic importance.

Spondyloarthropathies are a heterogeneous group of diseases sharing clinical and radiological manifestations, among which ankylosing spondylitis is the most prevalent. They are characterized by the presence of inflammation of the joints in the spine and sacroiliac joints, as well as their association with the presence of HLA-B27, which determines their familial association and a series of common pathogenic mechanisms. Although ankylosing spondylitis is less prevalent than RA, it also has a significant ability to cause the deterioration of patients' functional capacity which also entails high social costs derived particularly from work disability that they cause as it affects younger patients.

Additionally, systemic lupus erythematosus is a highly heterogeneous disease which is characterized by humoral immune system activation which determines the emergence of autoantibodies, commonly antinuclear antibodies, anti-DNA antibodies and others. Its clinical expression varies greatly from clinically mild and even silent forms to catastrophic forms affecting vital organs such as the kidney, brain, heart or lungs. It is less prevalent than the two IMIDs described above and mainly affects young women.

Evidence demonstrating that starting early treatment with disease-modifying drugs (DMDs) allows better IMID control has been accumulated in recent years. However, many patients fail to reach the ideal state of remission probably because a sufficiently intense treatment is not established from the start due to the inability to determine how severe the disease will become.

As a result, it is necessary to search for elements which allow determining how the disease will progress and therefore allow determining the treatment intensity suitable for each patient early on, taking the risks of more aggressive treatments for patients who would have a poor prognosis and avoiding these aggressive treatments in patients who would have a mild form of the disease. In this sense, some studies on the quantification of specific proteins or peptides in biological fluids, including VIP (for example, Martinez C *et al.* 2014 PLOS One 1:e85248), among others, have already been conducted; however, the quantification methods used up until now show poor inter-assay reproducibility, such that a more reliable and reproducible method is required. With respect to VIP, genetic variants associated with a higher risk of suffering from autoimmune diseases have not been observed. In fact, the genetic variants of specific molecules may have an impact on the risk of developing autoimmune diseases, but they may not influence the progression thereof and, in contrast, molecules the genetic variability of which does not influence the risk of developing an autoimmune disease may indeed modulate its severity. In this sense, studies on the genetic variants of various genes with association with the prognosis of RA have been conducted (for example WO2008010085), although there are no definitive results and, specifically, the *VIP* gene has not been previously studied from the viewpoint of prognosis prediction. Therefore, it is necessary to search for reproducible markers which allow improved determination of the prognosis of autoimmune diseases, or which can serve as alternatives to or complement the already available biomarkers for detecting patients who would respond better or worse to a specific treatment, which would allow time and cost savings in IMID management.

### DISCLOSURE OF THE INVENTION

In the present invention, genetic variants of the *VIP* gene which are associated with a higher risk of having a poor progression of autoimmune diseases, such as rheumatoid arthritis, spondyloarthritis and systemic lupus erythematosus, for example, and require a more intense treatment during follow-up, have been determined. Said genetic variants are associated with a low serum VIP peptide level.

In the present invention, the usefulness of several polymorphisms (single nucleotide polymorphisms, SNP) of the *VIP* gene, specifically SNPs rs35643203, rs71575932 and/or rs7755568, in the prognosis of rheumatoid arthritis is demonstrated. The authors have demonstrated that rs35643203, rs71575932 and rs7755568 are in linkage disequilibrium, so the three of them are the informative SNPs for the present invention. In addition to these SNPs, it has been demonstrated that if rs3823082 and/or rs688136 are used, the prognosis of the severity of the disease can also be made. Polymorphisms rs12213214, rs60946248, rs140023105, rs7764067, rs12201030, rs74760293, rs149081483 and rs12201140, or any of the combinations thereof, in combination with the preceding SNPs are also useful. Furthermore, the present invention shows results for other immune-mediated inflammatory diseases (IMID), such as spondyloarthritis and systemic lupus erythematosus, demonstrating that the results obtained for RA can be extrapolated to other IMIDs. The present invention therefore relates to a method for the prognosis of an autoimmune disease, such as inflammatory bowel disease, psoriasis, spondyloarthritis (for example, ankylosing spondylitis) or systemic lupus erythematosus, for example. It also relates to a method for establishing a customized treatment for a patient having an IMID.

A first aspect of the invention therefore relates to a method for obtaining data useful for the prognosis of an autoimmune disease which comprises detecting genetic polymorphisms rs35643203, rs71575932 and/or rs7755568 of the "vasoactive intestinal peptide" (*VIP*) gene in a biological sample isolated from a subject suffering from an autoimmune disease, which will be referred to hereinafter as the "first method of the invention."

In the present invention, "prognosis" is understood as the ability to determine in patients having an autoimmune disease how said disease will progress in terms of severity. The term "prognosis" also refers to the ability to detect subjects already diagnosed with an autoimmune disease with a high probability of the disease worsening. This poor progression or "poor prognosis" can be defined as a small possibility of achieving disease remission, taking into account that the person skilled in the art knows that there are many ways to define this ideal of remission in each disease. For example, in rheumatoid arthritis there are different ways to define the ideal state of remission, such as for example, but without limitation, the ways described in Felson et al. Arthritis Rheum 2011;63:573-586. Other situations which can also be considered a poor progression or poor prognosis are the failure to achieve a state of minimal disease activity (Wells et al. J Rheumatol 2005;32:2016-2024) or the possibility of having a poor therapeutic response either according to ACR20, 50 or 70 criteria (Felson et al. Arthritis Rheum 1995;38:727-735) or according to the European League Against Rheumatism's criteria (EULAR) (van Gestel et al. Arthritis Rheum 1996;39:34-40). In the present invention, a "poor prognosis" is also understood as having a higher probability of requiring a more intense treatment, for example as requiring a combined therapy of DMDs in the case of RA, regardless of whether or not one of the drugs is biological. Furthermore, a "poor prognosis" is also understood as an increase in the probability of greater joint destruction according to radiographic evaluation for the case of RA.

In the case of ankylosing spondylitis, a "poor prognosis" is considered as a failure to achieve the remission defined according to the cut-off points of the following indices for activity evaluation: BASDAI<2 (Juanola Roura X et al. Reumatol Clin 2011;7:113-23) or ASDAS<1.3 (Machado P et al. Ann Rheum Dis 2011;70:47-53). A "poor prognosis" is also understood as the need to start treatment with biological therapy or to have structural damage progression evaluated either by simple radiology or magnetic resonance.

For systemic lupus erythematosus, the definition of "poor prognosis," as is well known by the person skilled in the art, is more complex because this is a much more heterogeneous disease and clinical activity is measured more by outbreaks, having an SLEDAI index >3 (Calvo-Alén J et al. Reumatologia Clin 2013; 9: 281-296) being considered as a "poor prognosis," for example. Nevertheless, the severity of the disease can depend on the affected organ, such that while the disease is active, the forms of the disease affecting vital organs (heart, kidney, brain) would be more severe from the vital viewpoint than other forms affecting non-vital organs such as the skin or joints. Another way to evaluate the poor prognosis would be the refractoriness to various treatments and the requirement of more intense treatment.

A "good prognosis" is therefore understood as being contrary to everything described above, i.e., a higher probability of achieving a state of disease remission or minimal disease activity, either by being capable of achieving a suitable therapeutic response according to the criteria defined above or also by spontaneous disease remission.

The prognosis must preferably be made, although without limitation, in the first year of the onset of the symptoms of the autoimmune disease, even before the definitive diagnosis of the disease is established. Alternatively, the method can also be applied at any time during development of the disease.

In the present invention, "*VIP*" is understood as that gene having the capacity to encode the "vasoactive intestinal peptide" or "VIP." The gene can be, for example, although without limitation, the *VIP* gene with NCBI (*National Center for Biotechnology Information*) GenBank accession number AH003027. The VIP in the present invention relates to the peptide with NCBI GenBank accession number AAB22264.1 having amino acid sequence SEQ ID NO: 1 (HSDAVFTDNYTRLRKQMAVKKYLNSILN).

The term "genetic polymorphism" refers to a variation in the nucleotide sequence of a deoxyribonucleic acid (DNA) strand having at least a 1% frequency in individuals of a population. Genetic polymorphisms can be variations of one or more nucleotides. Single nucleotide polymorphism or SNP generally gives rise to two different alleles. SNPs or polymorphisms can contain information about variation of a single polymorphism or information about a haplotype if they are tag-SNPs. In the present invention, the terms "polymorphism" and "SNP" are used interchangeably.

In the present invention, it is demonstrated that polymorphisms rs35643203, rs71575932 and rs7755568 are in linkage disequilibrium, i.e., they are marker polymorphisms of a genomic region within a specific haplotype block.

"Haplotype" is understood as a combination of alleles in different loci of a chromosome which are transmitted together. A haplotype can be a locus, several loci, or an entire chromosome depending on the number of recombination events that have occurred between a given set of loci. Maternally transmitted alleles form the maternal haplotype, whereas paternal alleles form the paternal haplotype. It also refers to a set of polymorphisms (SNPs) which are inherited as a block with linkage disequilibrium measurements r²≥0,90 and identified by means of genotyping one of the polymorphisms included in said haplotype.

In the present invention, genotyped polymorphisms rs35643203, rs71575932 and rs7755568 belong to the same haplotype block according to the obtained results (i.e., they are tag-SNPs). Any genetic variant found within the same haplotype block as these genotyped tag-SNPs will be inherited together with them with a high probability.

Polymorphisms within a haplotype block are in linkage disequilibrium. "Linkage disequilibrium" (LD) is the property of some loci to not separate independently, i.e., they have a recombination frequency of less than 50%.

In the present invention, the term "minor allele" refers to the allele having the lowest representation in the population, as is known by the person skilled in the art, and therefore, the term "major allele" refers to the most frequent allele in the population.

Polymorphism rs35643203 is understood as the C>T substitution (in the complementary strand the substitution would be G>A) in position 153072433 located in intron 1 of the *VIP* gene.

Polymorphism rs71575932 is understood as the A>G substitution (in the complementary strand the substitution would be T>C) in position 153075844 located in intron 3 of the *VIP* gene.

Polymorphism rs7755568 is understood as the T>A substitution (in the complementary strand the substitution would be A>T) in position 153076955 located in intron 4 of the *VIP* gene.

In the present invention, the term "biological sample" refers to any sample which allows determining *VIP* polymorphisms in the individual from whom said sample has been obtained, and includes, but without limitation, biological fluids of an individual, obtained by means of any method known by a person skilled in the art which serves for such purpose. The biological sample comprises genomic deoxyribonucleic acid (DNA). The biological sample can be, for example, but without limitation, a fluid sample, such as blood, plasma, serum, saliva, urine, synovial fluid or lymph. It can also be a tissue sample. Other samples of interest can be, for example, peripheral blood mononuclear cells, for example, T-lymphocytes. The biological sample can furthermore originate from extractions routinely performed in analysis which can be performed periodically in patients.

The biological sample in the present invention can be fresh, frozen, fixed or fixed and embedded in paraffin.

In the present invention, the terms "subject," "individual" and "patient" are used interchangeably. The subject is preferably a human.

A preferred embodiment of the first aspect of the invention relates to the method which further comprises detecting genetic polymorphism rs3823082 and/or rs688136 of the *VIP* gene.

Polymorphism rs3823082 is understood as the C>T substitution (in the complementary strand the substitution would be G>A) in position 153074322 located in intron 2 of the *VIP* gene.

Polymorphism rs688136 is understood as the T>C substitution (in the complementary strand the substitution would be A>G) in position 153080061 located in exon 7 of the *VIP* gene corresponding to the 3' UTR region of its mRNA.

A more preferred embodiment of the first aspect of the invention relates to the method which further comprises detecting at least one of the genetic polymorphisms of the *VIP* gene selected from the list comprising: rs12213214, rs60946248, rs140023105, rs7764067, rs12201030, rs12201140, rs74760293 and rs149081483, or any of the combinations thereof, in a biological sample isolated from a subject.

Polymorphism rs12213214 is understood as the C>A substitution (in the complementary strand the substitution would be G>T) in position 153070786 located in the promoter of the *VIP* gene.

Polymorphism rs60946248 is understood as the C>T substitution (in the complementary strand the substitution would be G>A) in position 153071195 located in the promoter of the *VIP* gene.

Polymorphism rs140023105 is understood as the A>G substitution (in the complementary strand the substitution would be T>C) in position 153072056 located in exon 1 of the *VIP* gene corresponding with the 5' UTR region of its mRNA.

Polymorphism rs7764067 is understood as the A>T substitution (in the complementary strand the substitution would be T>A) in position 153074199 located in intron 2 of the *VIP* gene.

Polymorphism rs12201030 is understood as the A>G substitution (in the complementary strand the substitution would be T>C) in position 153076789 located in intron 4 of the *VIP* gene.

Polymorphism rs12201140 is understood as the A>T substitution (in the complementary strand the substitution would be T>A) in position 153076956 located in intron 4 of the *VIP* gene.

Polymorphism rs74760293 is understood as the T>C substitution (in the complementary strand the substitution would be A>G) in position 153077241 located in intron 4 of the *VIP* gene.

Polymorphism rs149081483 is understood as the T>G substitution (in the complementary strand the substitution would be A>C) in position 153078500 located in intron 6 of the *VIP* gene.

An even more preferred embodiment of the first aspect of the invention relates to the method where the autoimmune disease is preferably autoimmune arthritis, inflammatory bowel disease, psoriasis, spondyloarthritis or systemic lupus erythematosus. In an even more preferred embodiment, the autoimmune arthritis is recent-onset arthritis or rheumatoid arthritis.

In the present invention, an "autoimmune disease" is understood as that disease in which the cells of the immune system trigger a chronic inflammatory response in one or more tissues of the individual causing their deterioration or even destruction. In the present invention, the terms "autoimmune disease" and IMID are used interchangeably.

In the present invention, the term "autoimmune arthritis" would comprise both the terms "rheumatoid arthritis" and "undifferentiated arthritis" whether it is recent-onset or well established.

In the present invention, "recent-onset arthritis" (ROA) (or early arthritis) is understood as that disease consisting of inflammation of at least one joint, with less than one year of progression which meets the criteria pre-established by the American College of Rheumatology (ACR) and EULAR for "rheumatoid arthritis or RA" (Aletaha, Neogi et al. Ann Rheum Dis 2010;69:1580-1588), or which while not meeting said criteria does not meet the criteria of other autoimmune, degenerative or metabolic diseases that can explain the symptoms. The last case has been referred to as "undifferentiated arthritis" (UA) which end up resulting in RA in many cases when left to progress freely by itself. The definition of undifferentiated arthritis is progressively gaining acceptance among persons skilled in the art, since it is understood that the earlier the patients are subjected to treatment, the more options there will be for inducing remission and the establishment of immunomodulating treatment in patients who do not meet RA criteria is accepted today. In this invention, the terms ROA, RA or UA refer to a chronic and progressive systemic autoimmune disease, which causes chronic inflammation of essentially the joints, and which given its progressive nature, causes destruction of the joints with subsequent deformation and loss of functional capacity. Furthermore, this disease can cause extra-articular alterations in various organs. The disease activity can be determined by means of composite indices providing a number combining information on different clinical and analytical variables such as DAS28 (Prevoo et al. Arthritis Rheum 1995;38:44-48), SDAI, CDAI (Smolen et al. Rheumatology (Oxford) 2003;42:244-257) and others.

In the present invention, "spondyloarthritis" is understood as those autoimmune diseases with axial and/or peripheral manifestation meeting the classification criteria of the Assessment of SpondyloArthritis International Society (ASAS) (Rudwaleit et al. Ann Rheum Dis 2011;70:25-31).

In the present invention, "systemic lupus erythematosus" is understood as that autoimmune systemic disease defined by the criteria of the American College of Rheumatology (Tan et al. Arthritis Rheum·1982;25:1271-1277).

In the present invention, the term "inflammatory bowel disease" or "IBD" refers to chronic bowel inflammation in an individual, where said inflammation is due to the immune system of the individual. The two most common forms correspond to ulcerative colitis and Crohn's disease. Therefore in a preferred embodiment the autoimmune disease is ulcerative colitis or Crohn's disease.

In the present invention, "psoriasis" is understood as that skin disease characterized by poor functioning of the immune system, which causes excessive skin cell production. This disease gives rise to the formation of reddish patches covered in scales. Furthermore, excessive cell production also causes the infiltration of white cells into the skin. The lesions are generally located in regions with higher friction, such as for example, although without limitation, the elbows, knees or groin area.

Another even more preferred embodiment of the first aspect of the invention relates to the method where the biological sample is blood, serum, plasma, saliva, urine, synovial fluid or lymph.

Another even more preferred embodiment of the first aspect of the invention relates to the method where the subject is a human.

A second aspect of the present invention relates to an *in vitro* method for the prognosis of an autoimmune disease in a subject suffering from an autoimmune disease, which comprises:
a. detecting genetic polymorphisms rs35643203, rs71575932 and/or rs7755568 of the *VIP* gene in a biological sample ;
b. associating the T allele of genetic polymorphism rs35643203, associating the G allele of genetic polymorphism rs71575932 and/or associating the A allele of polymorphism rs7755568, with a poor prognosis.

This method will be referred to hereinafter as the "second method of the invention."

The term *"in vitro"* refers to the method of the invention being performed outside the body of the subject.

A preferred embodiment of the second aspect of the invention relates to the method which further comprises in step (a) detecting polymorphism rs3823082 and/or detecting polymorphism rs688136 of the *VIP* gene; and in step (b) associating the TT genotype of polymorphism rs3823082 with a poor prognosis and/or associating the CC genotype of polymorphism rs688136 with a good prognosis.

Another preferred embodiment of the second aspect of the invention relates to the *in vitro* method which further comprises detecting in step (a) at least one of the genetic polymorphisms of the *VIP* gene selected from the list comprising rs12213214, rs60946248, rs140023105, rs7764067, rs12201030, rs74760293, rs149081483 and rs12201140, or any of the combinations thereof, and associating in step (b) the A allele of polymorphism rs12213214, the T allele of polymorphism rs60946248, the G allele of polymorphism rs140023105, the T allele of polymorphism rs7764067, the C allele of polymorphism rs74760293, the G allele of polymorphism rs149081483 and the T allele of polymorphism rs12201140, or any of the combinations thereof, with a poor prognosis and/or associating the G allele of polymorphism rs12201030 with a good prognosis.

A more preferred embodiment of the second aspect of the invention relates to the *in vitro* method where the autoimmune disease is autoimmune arthritis, inflammatory bowel disease, psoriasis, spondyloarthritis or systemic lupus erythematosus. In an even more preferred embodiment, the autoimmune arthritis is recent-onset arthritis or rheumatoid arthritis. In another preferred embodiment, the inflammatory bowel disease is ulcerative colitis or Crohn's disease.

An even more preferred embodiment of the second aspect of the invention relates to the *in vitro* method where the biological sample is blood, serum, plasma, saliva, urine, synovial fluid or lymph.

Another even more preferred embodiment of the second aspect of the invention relates to the method where the subject is a human.

The methods of the invention allow obtaining information useful for making therapeutic decisions, which allows selecting patients with a higher probability of requiring an intense treatment. This results in a reduction of the direct and indirect costs of the disease and an improvement in the patient's quality of life.

The present invention also relates to a method according to the second method of the invention where if the subject has a poor prognosis after step (b), the subject is treated with a combined therapy of synthetic DMDs or of a synthetic DMD and a biological DMD, particularly if the biological therapy is a tumor necrosis factor (TNF) blocking agent. For example, but without limitation, the TNF blocking agent is adalimumab, certolizumab, etanercept, golimumab or infliximab. Synthetic DMDs can include, among others, azathioprine, anti-malarial drugs, methotrexate, leflunomide, sulfasalazine and/or cyclosporin A. In contrast, if the subject has a good prognosis after step (b), said subject is treated with a monotherapy of non-biological DMD.

A third aspect of the invention relates to an *in vitro* method for designing a personalized treatment for a subject suffering from an autoimmune disease which comprises detecting genetic polymorphisms rs35643203, rs71575932 and/or rs7755568 of the *VIP* gene in a biological sample, in which the presence of the T allele of genetic polymorphism rs35643203, the presence of the G allele of genetic polymorphism rs71575932 or the presence of the A allele of genetic polymorphism rs7755568 is indicative that the treatment to be administered is a combined therapy of synthetic and biological DMDs. This method will be referred to hereinafter as the "third method of the invention."

"Treatment" is understood as all the means used to cure or alleviate a disease. In the present invention, the treatment to be administered is a combined therapy of DMDs, where the drugs are at least one synthetic drug and at least one biological drug when the subject has a poor prognosis. The biological drug is preferably a tumor necrosis factor (TNF) blocking agent, for example, but without limitation, the TNF blocking agent is adalimumab, certolizumab, etanercept, golimumab or infliximab. Synthetic DMDs (also referred to as no-biological DMDs) can include, among others, azathioprine, anti-malarial drugs, methotrexate, leflunomide, sulfasalazine and/or cyclosporin A. In contrast, if the subject has a good prognosis, said subject is treated with a monotherapy of non-biological DMD.

A preferred embodiment of the third aspect of the invention relates to the *in vitro* method which further comprises detecting polymorphism rs3823082 and/or rs688136 and where the presence of the TT genotype of polymorphism rs3823082 is indicative that the treatment to be administered is said combined therapy and/or where the presence of the CC genotype of polymorphism rs688136 is indicative that the treatment to be administered is a monotherapy of synthetic DMD.

A more preferred embodiment of the third aspect of the invention relates to the *in vitro* method which further comprises detecting the polymorphism of the *VIP* gene selected from the list comprising rs12213214, rs60946248, rs140023105, rs7764067, rs12201030, rs12201140, rs74760293, rs149081483 and rs12201140, or any of the combinations thereof; and where the presence of the A allele of polymorphism rs12213214, the T allele of polymorphism rs60946248, the G allele of polymorphism rs140023105, the T allele of polymorphism rs7764067, the A allele of polymorphism rs7755568, the C allele of polymorphism rs74760293 and the G allele of polymorphism rs149081483 or any of the combinations thereof, is indicative that the treatment to be administered is said combined therapy and where the presence of the T allele of rs12201030 is indicative that the treatment to be administered is a monotherapy of synthetic DMD.

An even more preferred embodiment of the third aspect of the invention relates to the *in vitro* method where the autoimmune disease is autoimmune arthritis, inflammatory bowel disease, psoriasis, spondyloarthritis or systemic lupus erythematosus. In an even more preferred embodiment, the autoimmune arthritis is recent-onset arthritis or rheumatoid arthritis. In another preferred embodiment, the inflammatory bowel disease is ulcerative colitis or Crohn's disease.

Another even more preferred embodiment of the third aspect of the invention relates to the *in vitro* method where the biological sample is blood, serum, plasma, saliva, urine, synovial fluid or lymph.

The polymorphisms of the invention can be detected by means of any technique known by the person skilled in the art, for example, by sequencing, genotyping, amplification by means of polymerase chain reaction (PCR), restriction fragment length polymorphism (RFLP) or by means of restriction enzymes. The detection can performed by means of using of primers or probes.

As it is used herein, the term "sequencing" refers to the determination of the nucleotides of a template nucleic acid and of their order.

The conditions in which sequencing is performed generally include (a) contacting a template nucleic acid with a polymerase in a mixture further comprising a primer, a bivalent cation (for example, Mg²⁺), and nucleotides, generally, dNTPs and at least one ddNTP (dideoxynucleotide triphosphate), and (b) subjecting said mixture to a temperature sufficient for the polymerase to start incorporating the nucleotides into the primer by means of base complementarity with the template nucleic acid, and to give rise to a population of complementary DNA molecules of a different size. The separation of said population of complementary DNA molecules generally by means of electrophoresis allows determining the nucleotide sequence.

As it is used herein, the term "amplification" refers to the increase in the number of template nucleic acid copies.

The conditions in which amplification is performed generally include (a) contacting a template nucleic acid with a polymerase in a mixture further comprising at least one primer (normally two primers), a bivalent cation (for example, Mg²⁺), and nucleotides, generally, dNTPs (b) subjecting said mixture to a temperature sufficient for the polymerase to start incorporating the nucleotides into the primer by means of base complementarity with the template nucleic acid, and to give rise to a population of complementary DNA molecules generally of the same size.

The PCR also can be real time quantitative PCR.

As it is used herein, the term "template nucleic acid" or "template" refers to a single-stranded or double-stranded nucleic acid molecule that will be amplified or sequenced.

As it is used herein, the term "primer" (also referred to as "oligo") refers to an oligonucleotide capable of acting as the starting point of DNA synthesis when it hybridizes with the template nucleic acid. The primer is preferably a deoxyribose oligonucleotide. Primers can be prepared by means of any suitable method, including, for example, but without limitation, cloning and restriction of suitable sequences and direct chemical synthesis. Primers can be designed to hybridize with specific nucleotide sequences in the template nucleic acid (specific primers) or can be randomly synthesized (arbitrary primers).

According to the present invention a "primer" can be tagged or labeled by means of techniques well known in the prior art. Detectable labels include, for example, radioactive isotopes, fluorescent labels, chemiluminescent labels, bioluminescent labels or enzyme labels.

In a preferred embodiment, the primers are those described as SEQ ID NO: 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or any of the combinations thereof. Furthermore, the primers can be modified to comprise sequences useful in the technique used, for example, in sequencing. For example, the primers can comprise the sequence SEQ ID NO: 60 bound to their 5' end in the case of the forward primer, and the sequence SEQ ID NO: 61 bound to the 5' end of the reverse primer.

The methods of the invention can be applied at any time during the development of the autoimmune disease. The methods of the invention can also be applied in patients not treated with immunomodulating drugs.

The methods of the invention can further comprise quantifying at least one *VIP* gene expression product in an isolated biological sample and/or also quantifying clinical variables. These clinical variables can be, for example, although without limitation, the sex of the patient, the state of activity at disease onset, as well as other variables known by the person skilled in the art. If recent-onset arthritis or rheumatoid arthritis is being studied, said variables can be anti-citrullinated peptide antibodies (ACPAs). So a particular embodiment of the first, second and third aspects of the invention relates to methods where ACPAs are furthermore detected. ACPAs can be detected by techniques known by the person skilled in the art, such as ELISA, for example.

All the steps of the methods of the invention can be fully or partially automated. In addition to the steps specified above, the methods described in the present description can comprise other additional steps relating, for example, to pretreatment of the sample or to extraction of the genetic material required for subsequent analysis.

A fourth aspect of the invention relates to the use of genetic polymorphism rs35643203, rs71575932 and/or rs7755568 of the *VIP* gene as a prognostic marker of an autoimmune disease in a subject suffering from an autoimmune disease.

A preferred embodiment of the fourth aspect of the invention relates to the use further comprising the use of genetic polymorphism rs3823082 and/or rs688136 of the VIP gene.

A more preferred embodiment of the fourth aspect of the invention relates to the use further comprising the use of the genetic polymorphisms of the *VIP* gene selected from the list comprising: rs12213214, rs60946248, rs140023105, rs7764067, rs12201030, rs74760293, rs149081483 and rs122001140, or any of the combinations thereof.

An even more preferred embodiment of the fourth aspect of the invention relates to the use where the autoimmune disease is autoimmune arthritis, inflammatory bowel disease, psoriasis, spondyloarthritis or systemic lupus erythematosus. In an even more preferred embodiment, the autoimmune arthritis is recent-onset arthritis or rheumatoid arthritis. In another preferred embodiment, the inflammatory bowel disease is ulcerative colitis or Crohn's disease.

A fifth aspect of the invention relates to a kit comprising primers or probes for detecting genetic polymorphisms rs35643203, rs71575932 and/or rs7755568 of the *VIP* gene.

A preferred embodiment of the fifth aspect of the invention relates to the kit further comprising primers or probes for detecting genetic polymorphisms rs3823082 and/or rs688136 of the *VIP* gene.

A more preferred embodiment of the fifth aspect of the invention relates to the kit further comprising primers or probes detecting genetic polymorphisms rs12213214, rs60946248, rs140023105, rs7764067, rs12201030, rs74760293, rs149081483 and rs12201140 of the *VIP* gene, or any of the combinations thereof.

Another even more preferred embodiment of the fifth aspect of the invention relates to the kit further comprising the elements required for detecting ACPAs, for example, primers, probes, specific antibodies, citrullinated peptides or citrullinated proteins.

In a preferred embodiment, the kit comprises the primers described as SEQ ID NO: 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or any of the combinations thereof. Furthermore, the primers can be modified to comprise sequences useful in the technique used, for example, in sequencing. For example, the primers can comprise the sequence SEQ ID NO: 60 bound to their 5' end in the case of the forward primer, and the sequence SEQ ID NO: 61 bound to the 5' end of the reverse primer.

The kit of the invention can further comprise at least one DNA polymerase, a reverse transcriptase, an RNA polymerase or a fluorophore. Furthermore, the kit can comprise a mixture of deoxynucleotide triphosphates (dNTPs), a mixture of nucleotide triphosphates (NTPs), deoxyribonuclease (DNase), ribonuclease (RNase) inhibitors, Dithiothreitol (DTT), inorganic pyrophosphatase (iPP) and the buffers required for the enzymes provided in the kit. The kits can further contain other molecules, genes, proteins or probes of interest, serving as positive and negative controls. The kits preferably further comprise instructions for carrying out the methods of the invention.

In the present invention, the probe or primers can be located in a solid support, for example, but without limitation, glass, plastic, tubes, multiwell plates, membranes, or any other known support.

A sixth aspect of the invention relates to the use of the kit of the fifth aspect of the invention for the prognosis of an autoimmune disease in a subject suffering from said disease.

A preferred embodiment of the sixth aspect of the invention relates to the use where the autoimmune disease is autoimmune arthritis, inflammatory bowel disease, psoriasis, spondyloarthritis or systemic lupus erythematosus. In an even more preferred embodiment, the autoimmune arthritis is recent-onset autoimmune arthritis or rheumatoid arthritis. In another preferred embodiment, the inflammatory bowel disease is ulcerative colitis or Crohn's disease.

It can be deduced from the present invention that it also relates to the methods of the invention when the detected polymorphisms are polymorphisms linked to the major alleles described in the present invention. The same occurs in the case of using said polymorphisms of the fourth aspect of the invention, the kit of the fifth aspect of the invention and using the kit of the sixth aspect of the invention.

The terms "polynucleotide," "nucleotide sequence," "nucleic acid" and "oligonucleotide" are used interchangeably in the present invention to refer to a polymeric nucleotide form of any length which may or may not be chemically or biochemically modified.

Throughout the description and the claims the word "comprises" and variants thereof do not intend to exclude other technical features, supplements, components or steps. For persons skilled in the art, other objects, advantages and features of the invention will be inferred in part from the description and in part from the practice of the invention. The following examples and drawings are provided by way of illustration and are not meant to limit the present invention.

### BRIEF DESCRIPTION OF THE DRAWINGS

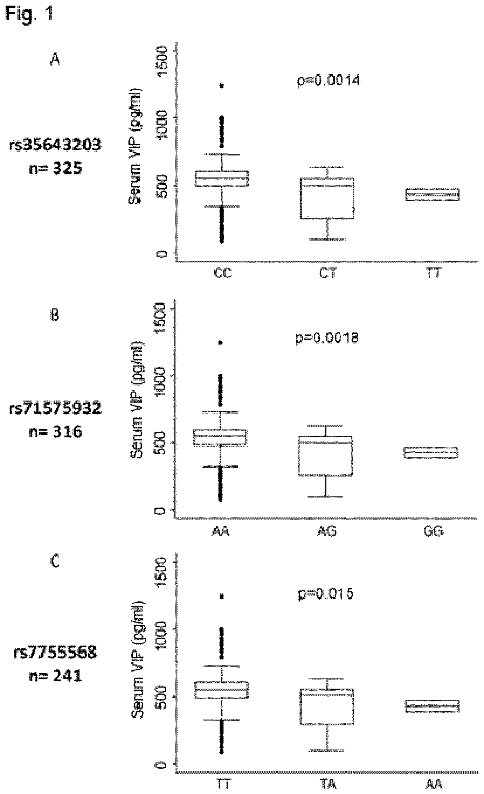

Figure 1 depicts the serum VIP levels in patients with early arthritis according to the genotype of polymorphisms rs35643203 (panel A), rs71575932 (panel B) and rs7755568 (panel C).

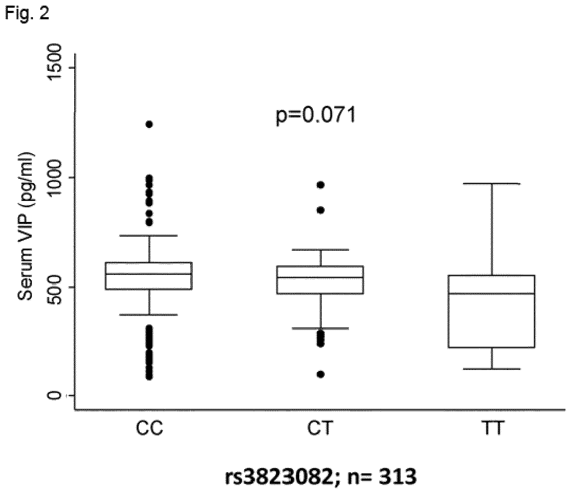

Figure 2 depicts the serum VIP levels according to the genotype of polymorphism rs3823082 in RA patients.

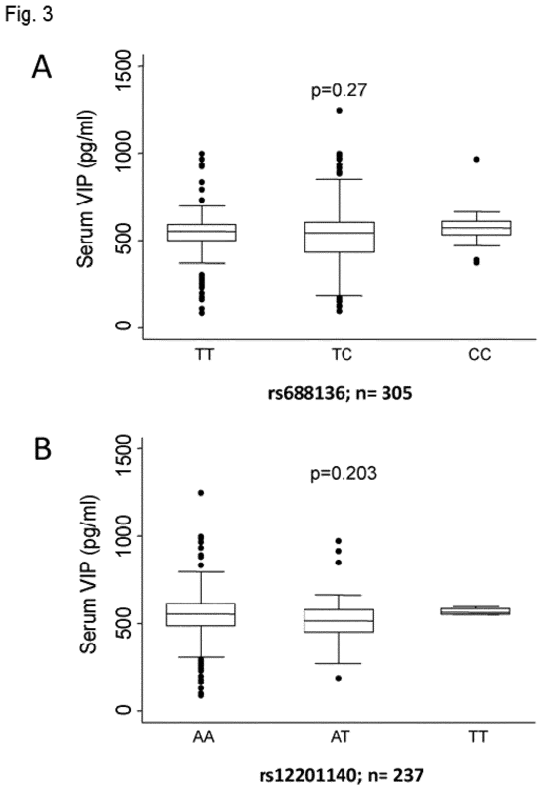

Figure 3 depicts the serum VIP levels according to the genotype of polymorphism rs688136 (panel A) and rs12201140 (panel B) in RA patients.

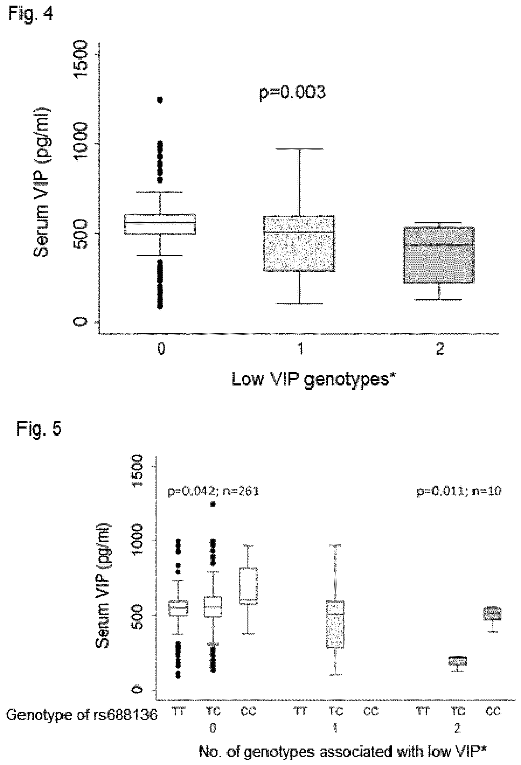

Figure 4 depicts the serum VIP levels in RA patients according to the combination of genotypes of rs3823082, rs35643203, rs71575932 and rs7755568. *On the x-axis, label 0 corresponds to not being homozygous for T of rs3823082 and not having any minor allele of rs35643203, rs71575932 or rs7755568. Label 1 corresponds to being homozygous for T of rs3823082 or having at least one minor allele of rs35643203, rs71575932 or rs7755568. Label 2 corresponds to being homozygous for T of rs3823082 and having at least one minor allele of rs35643203, rs71575932 or rs7755568.
Figure 5 depicts how the presence of genotypes associated with low VIP levels and the CC genotype of rs688136 which is associated with higher VIP levels interfere with one another. *On the x-axis, label 0 corresponds to not being homozygous for T of rs3823082 and not having any minor allele of rs35643203, rs71575932 or rs7755568. Label 1 corresponds to being homozygous for T of rs3823082 or having at least one minor allele of rs35643203, rs71575932 or rs7755568. Label 2 corresponds to being homozygous for T of rs3823082 and having at least one minor allele of rs35643203, rs71575932 or rs7755568.

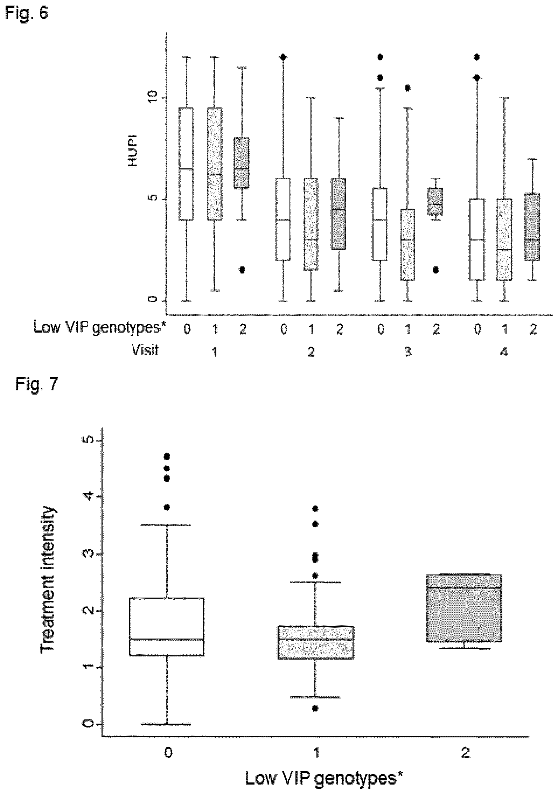

Figure 6 depicts levels of activity (estimated with the Hospital Universitario La Princesa index [HUPI]; Castrejon et al, Arthritis Care Res 2013; 65: 518-25) throughout the follow-up process according to the combination of genotypes of rs3823082, rs35643203, rs71575932 and rs7755568 which are associated with RA patients having a low serum VIP. *On the x-axis, label 0 corresponds to not being homozygous for T of rs3823082 and not having any minor allele of rs35643203, rs71575932 or rs7755568. Label 1 corresponds to being homozygous for T of rs3823082 or having at least one minor allele of rs35643203, rs71575932 or rs7755568. Label 2 corresponds to being homozygous for T of rs3823082 and having at least one minor allele of rs35643203, rs71575932 or rs7755568.
Figure 7 depicts treatment intensity after two years of follow-up (calculated as the number of days with DMD in the two years of follow-up/number of days between baseline and final visit; for a more comprehensive definition, see Gonzalez-Alvaro PLoS One 2011; 6: e29492), according to the combination of genotypes of rs3823082, rs35643203, rs71575932 and rs7755568 which are associated with RA patients having a low serum VIP. *On the x-axis, label 0 corresponds to not being homozygous for T of rs3823082 and not having any minor allele of rs35643203, rs71575932 or rs7755568. Label 1 corresponds to being homozygous for T of rs3823082 or having at least one minor allele of rs35643203, rs71575932 or rs7755568. Label 2 corresponds to being homozygous for T of rs3823082 and having at least one minor allele of rs35643203, rs71575932 or rs7755568.

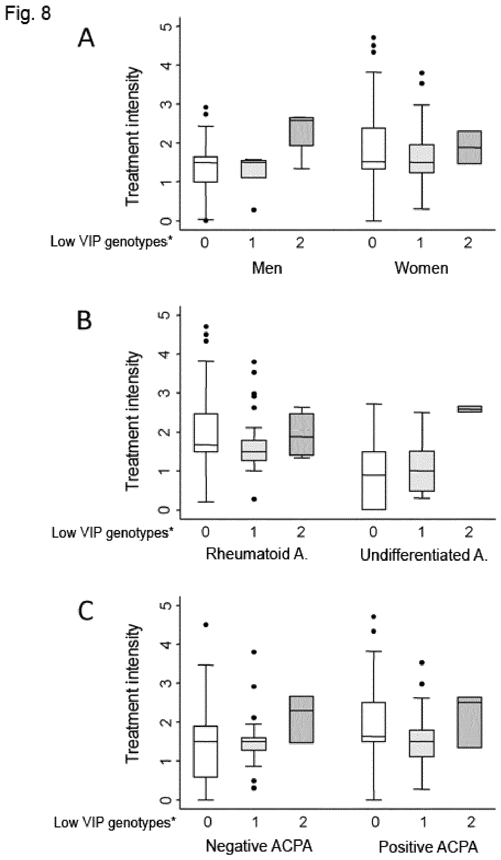

Figure 8 depicts treatment intensity after two years of follow-up (calculated as the number of days with DMD in the two years of follow-up/number of days between baseline and final visit; for a more comprehensive definition, see Gonzalez-Alvaro PLoS One 2011; 6: e29492), according to the combination of genotypes of rs3823082, rs35643203, rs71575932 and rs7755568 which are associated with having a low serum VIP stratified by gender (A), diagnosis (B) and result of the anti-citrullinated protein antibody (ACPA; C) assay in RA patients. *On the x-axis, label 0 corresponds to not being homozygous for T of rs3823082 and not having any minor allele of rs35643203, rs71575932 or rs7755568. Label 1 corresponds to being homozygous for T of rs3823082 or having at least one minor allele of rs35643203, rs71575932 or rs7755568. Label 2 corresponds to being homozygous for T of rs3823082 and having at least one minor allele of rs35643203, rs71575932 or rs7755568.

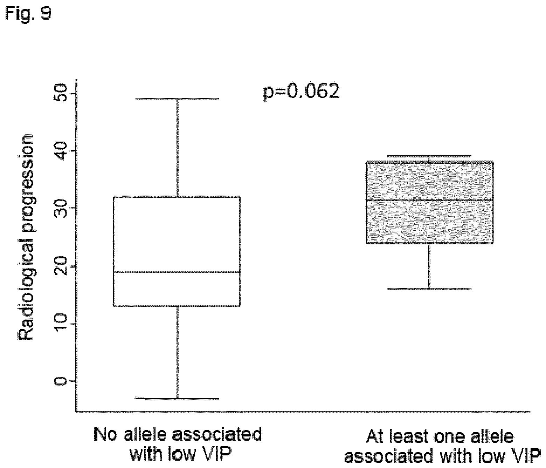

Figure 9 depicts radiological progression after two years of follow-up according to whether or not the patients have one of the genotypes of rs3823082, rs35643203, rs71575932 or rs7755568 which are associated with RA patients having a low serum VIP.

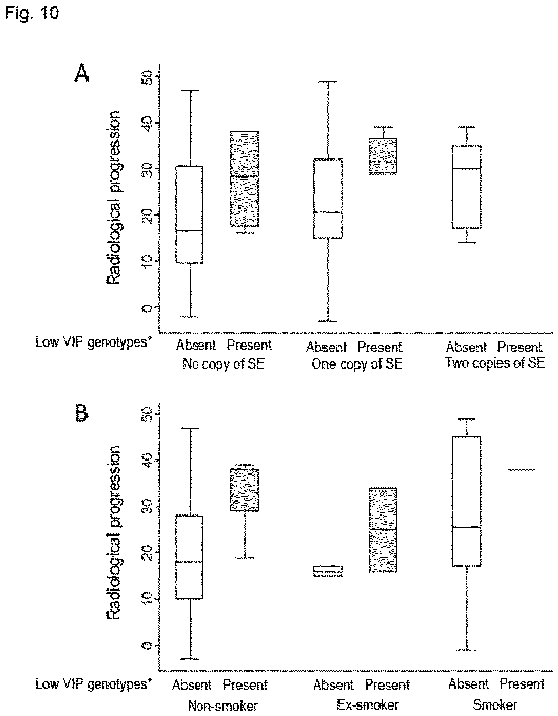

Figure 10 depicts radiological progression after two years of follow-up according to whether or not the patients have one of the genotypes of rs3823082, rs35643203, rs71575932 or rs7755568 which are associated with RA patients having a low serum VIP, stratified by number of copies of the shared epitope (A) or of the smoking habit (B).

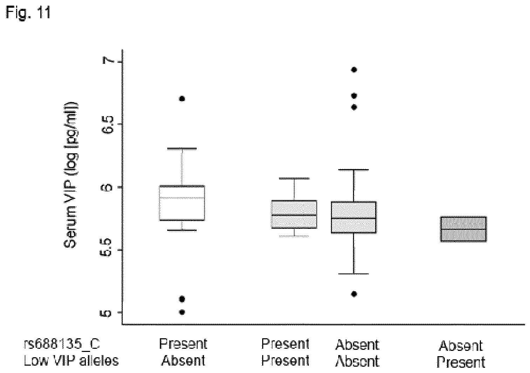

Figure 11 depicts how the presence of genotypes associated with low VIP levels in rheumatoid arthritis patients (being homozygous TT of rs3823082 or having at least one minor allele of rs35643203, rs7157932 or rs7755568) interferes with having a C allele of rs688136 in the serum VIP levels of patients from the early spondyloarthritis register.

### EXAMPLES

The invention will be illustrated below by means of assays performed by the inventors, which clearly show the usefulness of the methods of the invention. These examples are included only for illustrative purposes and must not be interpreted as limiting the invention claimed herein. Therefore, the examples described below illustrate the invention without limiting the field of application thereof.

### Example 1: Descriptive study of VIP genetic variants linked to VIP serum levels in rheumatoid arthritis

The *VIP* gene was sequenced, and it was surprisingly found that specific genetic variants of the *VIP* gene correlated with low serum VIP peptide levels, and it was demonstrated that specific genotypes appeared in patients with a poorer prognosis (i.e., poorer progression or greater severity) and need for a more intense treatment.

Two types of studies were carried out: in the first study, 10 patients with low serum VIP levels and 15 patients with high serum VIP levels were selected for whole VIP gene sequencing and detecting the differences existing between the two groups; in the second study, the genetic variants detected in the first study in the entire population with early arthritis of Hospital Universitario (HU) La Princesa (342 patients with at least two years of follow-up) were validated.

Population: for this study, out of 91 patients whose VIP levels were determined during follow-up in the study, 10 patients were selected whose VIP levels throughout the follow-up process were below the 25^{th} percentile of the control population (100 healthy blood donors from Centro Nacional de Transfusiones, Madrid). Out of the 10 patients, 5 patients had VIP levels below the 10^{th} percentile. Additionally, 15 patients with VIP levels above the 90^{th} percentile of the control population were selected. DNA was obtained from these 25 patients and the VIP gene was sequenced from -1933 base pairs (bp) before the start of transcription to 2651 bp after the end of the last exon.

The gene was split into 29 overlapping segments (amplicons) for sequencing. For most of the amplicons, the primer sequences described by Applied Biosystems® and accessible through the NCBI were used (">gnI|Probe|1357097b.1 RSA probe RSA001033793, PCR primer (42 bp); >gnI|Probe|1357097c.1 RSA probe RSA001033793, PCR primer (44 bp); >gnI|Probe|1357099b.1 RSA probe RSA001033798, PCR primer (38 bp); >gnI|Probe|1357100b.1 RSA probe RSA001033801, PCR primer (38 bp); >gnI|Probe|1357101b.1 RSA probe RSA001033803, PCR primer (38 bp); >gnI|Probe|1361007c.1 RSA probe RSA000585838, PCR primer (38 bp); >gnI|Probe|1357100c.1 RSA probe RSA001033801, PCR primer (39 bp); >gnI|Probe|1357101c.1 RSA probe RSA001033803, PCR primer (44 bp); >gnI|Probe|1357102b.1 RSA probe RSA001033807, PCR primer (38 bp); >gnI|Probe|1357103b.1 RSA probe RSA001033810, PCR primer (44 bp); >gnI|Probe|1357103c.1 RSA probe RSA001033810, PCR primer (42 bp); >gnI|Probe|1357317c.1 RSA probe RSA001031301, PCR primer (42 bp); >gnI|Probe|1357318b.1 RSA probe RSA001031306, PCR primer (43 bp); >gnI|Probe|1357318c.1 RSA probe RSA001031306, PCR primer (44 bp); >gnI|Probe|1356759b.1 RSA probe RSA001046167, PCR primer (37 bp); >gnI|Probe|1357310b.1 RSA probe RSA001031315, PCR primer (44 bp); >gnI|Probe|1356759c.1 RSA probe RSA001046167, PCR primer (38 bp); >gnI|Probe|1357311b.1 RSA probe RSA001031320, PCR primer (42 bp); >gnI|Probe|1357313b.1 RSA probe RSA001031334, PCR primer (44 bp); >gnI|Probe|1356753c.1 RSA probe RSA001046172, PCR primer (38 bp)," referring to sequences SEQ ID NO: 2, 3, 4, 5, 6, 7, 8, 10, 14, 21, 24, 25, 27, 29, 30, 32, 33, 35, 36, 37, 38, 39, 40, 41, 42, 44, 57 and 59). Nevertheless, there was a need to design new specific primers to cover areas that are not described (mainly in introns) and to even split some of the described amplicons due to their large size (SEQ ID NO: 9, 11, 12, 13, 15, 16, 17, 18, 19, 20, 22, 23, 26, 28, 31, 34, 43, 45, 46, 47, 48, 49, 50, 51, 52, 53, 54, 55, 56, 58). The list of (forward and reverse) primers for the different amplicons used is shown in sequences SEQ ID NO: 2 to 59. To prevent sequencing errors, primers were also used in which at their 5' end the sequence TGTAAAACGACGGCCAGT was added in the forward primers (SEQ ID NO: 60) and the sequence CAGGAAACAGCTATGACC was added in the reverse primers (SEQ ID NO: 61), where said sequences refer to an added sequence of the M13 virus used for verifying the correct sequencing. Sequencing was carried out in a capillary electrophoresis genetic analyzer with 8 capillaries of Applied Biosystems® (3500 Genetic Analyzer) which allows re-sequencing the gene to search for mutation profiles using only one polymer in the capillary array.

High quality sequencing data was obtained in 9 patients with low VIP and 11 patients with high VIP. The main clinical data of these patients according to their VIP expression level (high or low) is shown in Table 1. Once the sequences of the 29 amplicons for each of the 20 patients were obtained, they were compared with the consensus VIP sequence published in PubMed with the reference NC_000006.11 (Chromosome 6; 153071932-153080900) by means of the Variant Reporter software (Applied Biosystems®). In this process, 17 single nucleotide polymorphisms (SNPs) were detected, the 15 described above and 2 new ones, with respect to the consensus VIP sequence. Table 2 describes in more detail the genetic variants detected in the 20 patients with their distribution according to serum VIP levels.

**Table 1. Clinical data of patients in whom VIP sequencing was performed according to the serum levels of this neuropeptide.**

| | High VIP (n=11) | Low VIP (n=9) | Total (n=20) | P |
|---|---|---|---|---|
| Female | 8 (73%) | 5 (56%) | 13 (65%) | 0,37 |
| Age (med [p25, p75]) | 58 [49 - 69] | 46 [41 - 54] | 54 [46 - 64] | 0.04 |
| Diagnosis | | | | |
| Rheumatoid arthritis | 7 (64%) | 7 (78%) | 14 (70%) | 0.43 |
| Undifferentiated arthritis | 4 (36%) | 2 (22%) | 6 (30%) | |
| Rheumatoid Factor + | 3 (27%) | 4 (44%) | 7 (35%) | 0.37 |
| ACPA + | 1 (9%) | 5 (56%) | 6 (30%) | 0.05 |
| Smoker | 4 (40%) | 4 (50%) | 8 (45%) | 0.80 |
| Baseline DAS28 (med [p25, p75]) | 5.7 [4.4 - 6.8] | 4.6 [4.4 - 4.8] | 4.8 [4.4 - 6.4] | 0.19 |
| Baseline HUPI (med [p25, p75]) | 9.5 [7 - 11.5] | 7 [6.5 - 8] | 8 [6.5 - 11.5] | 0.41. |

| | | | | |
|---|---|---|---|---|
| ACPA: anti-citrullinated protein antibodies; DAS28: 28-joint count disease activity score; HUPI: HU La Princesa activity index (Castrejon et al. Arthritis Care Res 2013, 65(4):518-25); med: median; p25: 25^{th} percentile; p75: 75^{th} percentile; p≤0.05 was considered significant. | | | | |

As seen in Table 2, out of the 17 SNP-type variants, four showed almost significant differences between the two groups of patients (high VIP and low VIP): rs35643203, rs3823082, rs71575932 and rs7755568.

rs35643203: relates to a C>T substitution (in the complementary strand the substitution would be G>A) in position 153072433 located in intron 1 of the *VIP* gene. The minor allele frequency (MAF) of allele T in the general population is 3% and in the present invention a MAF of 17% was found in patients with low VIP and of 0% in patients with high VIP. This position was correctly sequenced in the 20 patients and could furthermore be confirmed as it was covered by amplicons 4 and 5.

rs3823082: relates to a C>T substitution (in the complementary strand the substitution would be G>A) in position 153074322 located in intron 2. The minor allele frequency of allele T described in the general population is 25%. In the present invention, a MAF of 28% was found in patients with low VIP and of 4% in patients with high VIP. This position was correctly sequenced in the 20 patients, being located in amplicon 9.

rs71575932: relates to an A>G substitution (in the complementary strand the substitution would be T>C) in position 153075844 located in intron 3. The minor allele frequency of allele G described in the general population is 3%. In the present invention, a MAF of 17% was found in patients with low VIP and of 0% in patients with high VIP. This position was correctly sequenced in the 20 patients, with confirmation as it was represented in amplicons 14 and 15.

rs7755568: relates to a T>A substitution (in the complementary strand the substitution would be A>T) in position 153076955 located in intron 4. The minor allele frequency of allele A described in the general population is 8%. In the present invention, a MAF of 22% was found in patients with low VIP and of 0% in patients with high VIP. This position was correctly sequenced in the 20 patients, being located in amplicon 17.

**Table 2: Genetic variants detected in patients with recent-onset arthritis according to VIP expression**

| Cluster report | Position acc. to NC_000006.11:g | Position acc. to NM_003381.3:c. | Region | MAF | High VIP | Low VIP | p |
|---|---|---|---|---|---|---|---|
| rs12213214 | 153070786C>A | -1319C>A | Promoter | 0.223 | 1 CA (9%) | 2 CA (22%) | 0.421 |
| rs60946248 | 153071195C>T | -910C>T | Promoter | 0.025 | 0 (0%) | 1 CT (11%) | 0.45 |
| rs140023105 | 153072056A>G | -49A>G | 5' UTR Exon 1 | 0.09 | 0 (0%) | 1 AG (11%) | 0.474 |
| **rs35643203** | 153072433C>T | -11+339C>T | Intron 1 | 0.033 | 0 (0%) | 3 CT (33%) | 0.074 |
| rs3799142 | 153072696T>C | -11+602T>C | Intron 1 | 0.17 | 1 TC (9%) | 1 TC (11%) | 0.711 |
| rs7764067 | 153074199A>T | 107+780A>T | Intron 2 | 0.209 | 1 AT (9%) | 2 AT (22%) | 0.421 |
| **rs3823082** | 153074322C>T | 107+903C>T | Intron2 | 0.255 | 1 CT (9%) | 3 CT (33%) | 0.166 |
| | | | | | | 1 TT (11%) | |
| New | 153074732A>G | | Intron 2 | ? | 0 AG (0%) | 1 AG (11%) | 0.45 |
| rs12201173 | 153075076T>C | 108-225T>C | Intron 2 | 0.155 | 1 TC (9%) | 1 TC (11%) | 0.71 |
| **rs71576932** | 153075844A>G | 230+421A>G | Intron3 | 0.034 | 0 AG (0%) | 3 AG (33%) | |
| rs12201030 | 153076789A>G | 335+281A>G | Intron 4 | 0.089 | 2 AG (18%) | 1 AG (11%) | 0.579 |
| **rs7765568** | 153076955T>A | 336-314T>A | Into 4 | 0.080 | 0 TA (0%) | 2 TA (22%) 1 AA (11%) | 0.074 |
| | | | | | | | |
| rs12201140 | 153076956A>T | 336-313A>T | Intron 4 | 0.142 | 0 AT (0%) | 1 AT (11%) | 0.45 |
| rs74760293 | 153077241T>C | 336-28T>C | Intron 4 | 0.042 | 0 TC (0%) | 1 TC (11%) | 0.45 |
| rs149081483 | 153078500T>G | '43+181T>G | Intron 6 | <0,01 | 0 TG (0%) | 1 TG (12%) | 0.471 |
| *New* | 153078574C>A | | Intron 6 | ? | 1 CA (11%) | 0 CA (0%) | 0.529 |
| rs688136 | 153080061T>C | '60T>C | 3'UTR | 0.357 | 2 TC (16%) | 4 TC (44%) | 0.448 |
| | | | Exon 7 | | 2 CC (18%) | | |

| | | | | | | | |
|---|---|---|---|---|---|---|---|
| "?" refers to the MAF in the general population being unknown as it is a new description | | | | | | | |

### Example 2: Validation of VIP single nucleotide polymorphisms rs3823082, rs7755568, rs35643203, rs71575932 and rs688136

Out of the 17 genetic variants described with respect to the consensus sequence (Table 2), in a first step the decision was made to study those SNPs showing a different distribution between patients with low VIP levels and patients with high VIP levels with a statistically significant trend. Given the small number of patients being studied, it was considered that p<0.2 can be one way to prioritize the study of the genetic variants. Furthermore, SNP rs688136 was studied as it is the only polymorphism the minor variant of which was more widely represented in patients with high VIP. For the validation study, 457 patients from the recent-onset arthritis register of the HU La Princesa with available DNA samples were used (complete clinical data in Table 3). In the case of SNPs rs3823082, rs35643203, rs71575932 and rs688136, genotyping was performed by means of TaqMan® probes of Applied Biosystems® (catalog numbers: C_27491244_10, C_3250637_10, C_3250638_10 and C_3250639_10, respectively, of the reference TaqMan Genotyping Assays 4351379). In the case of SNP rs7755568, genotyping was carried out by means of sequencing amplicon 17 as described above. This allowed obtaining information of the other two genetic variants also present in said amplicon: rs12201030 and rs12201140.

**Table 3. Clinical data of patients in whom the validation of the data obtained in Example 1) was performed.**

| | Total (n=457) | With VIP in serum (n=87) | With 2-year follow-up (n=307) |
|---|---|---|---|
| Female | 365 (80%) | 63 (72%) | 240 (78%) |
| Age (med [p25, p75]) | 54 [42 - 66] | 54 [44 -66] | 54 [43 - 66] |
| Diagnosis Rheumatoid arthritis | 276 (60%) | 66 (77%) | 216 (70%) |
| Undifferentiated arthritis | 169 (37%) | 21 (23%) | 91 (30%) |
| Others | 12 (3%) | | |
| Rheumatoid Factor + | 201 (44%) | 38 (44%) | 152 (49,5%) |
| ACPA + | 182 (40%) | 42 (48%) | 142 (46%) |
| Smoker | 193 (44%) | 40 (46%) | 122 (42%) |
| Baseline DAS28 (med [p25, p75]) | 4.3 [3.3 - 5.5] | 4.9 [3.6 - 6] | 4.5 [3.5 - 5.7] |
| Baseline HUPI (med [p25, p75]) | 6.5 [4 - 9.5] | 8 [6 -10] | 7 [4.5 - 10] |

| | | | |
|---|---|---|---|
| ACPA: anti-citrullinated protein antibodies; DAS28: 28-joint count Disease Activity Score; HUPI: HU La Princesa activity index (Castrejon et al. Arthritis Care Res 2013, 65(4):518-25); med: median; p25: 25^{th} percentile; p75: 75^{th} percentile. | | | |

As quality control, the genotype obtained by sequencing for SNPs rs3823082, rs35643203, rs71575932 and rs688136 in the 20 patients of Example 1) was confirmed by means of genotyping using quantitative PCR and TaqMan probes. Overall, the genotype of the first 2 SNPs was obtained in 440 patients on the register, in 428 patients in the case of rs71575932 and in 425 patients for rs688136. The MAF (minor allele frequency, in this case the T allele) of rs3823082 is 23.2%. The MAF (T allele) of rs35643203 is 6.5%. The MAF of rs71575932 (G allele) is 7.5%. The MAF (C allele) of rs688136 is 34%.

In the case of rs7755568, this SNP could not be genotyped by means of TaqMan probes because it is located in a region predominantly having bases A and T, making it impossible to obtain primers that are specific enough. For this reason, the decision was made to perform genotyping by means of sequencing amplicon 17, which allowed knowing the genotype of rs12201030 and rs12201140 as well. The genotype of 293, 301 and 291 patients, respectively, was obtained. The MAF (A allele) of rs7755568 is 6.8%. The MAF (G allele) of rs12201030 is 10.6%, and the MAF (T allele) of rs12201140 is 12.9%.

As seen in the following expression tables, some of these 7 genetic variants have a significant association that almost reaches perfect linkage disequilibrium between rs35643203, rs71575932 and rs7755568. Although these three genetic variants are located in different introns (1, 3 and 4), as seen in Tables 4, 5 and 6, their alleles were distributed in a virtually identical manner. So it can be concluded that SNPs rs35643203, rs71575932 and rs7755568 are in linkage disequilibrium.

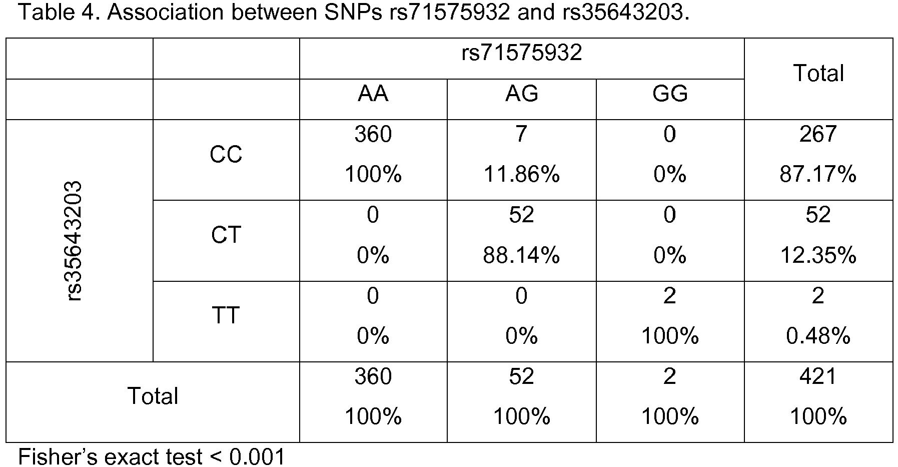

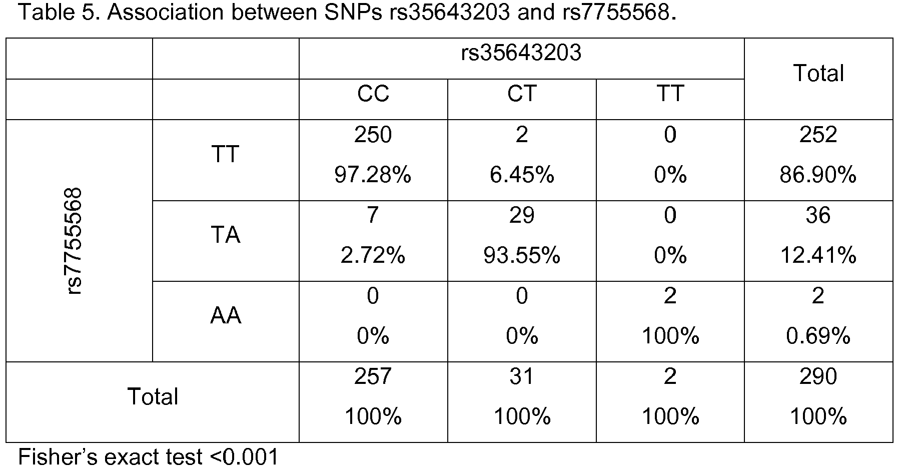

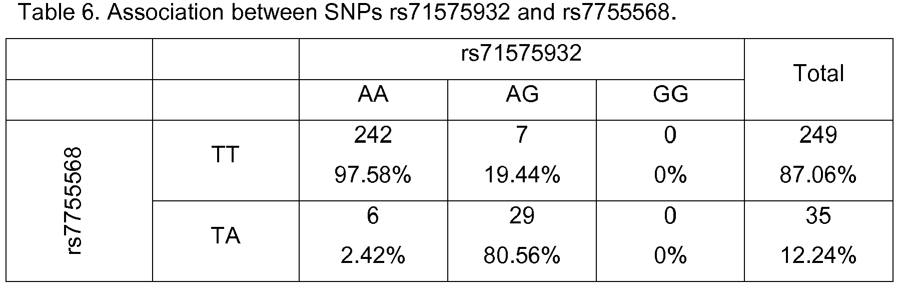

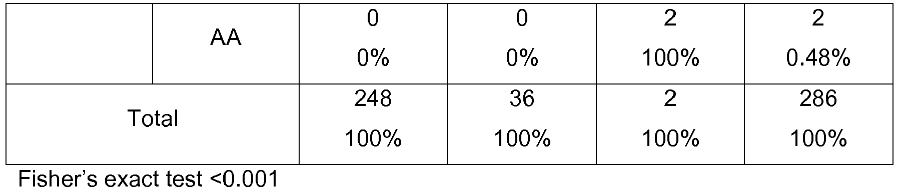

To demonstrate that the association is not as perfect with the other SNPs (rs3823082, rs688136, rs12201030 and rs12201140), rs35643203 has been chosen as the representative of the 3 preceding SNPs. Even with some of them the association is insignificant.

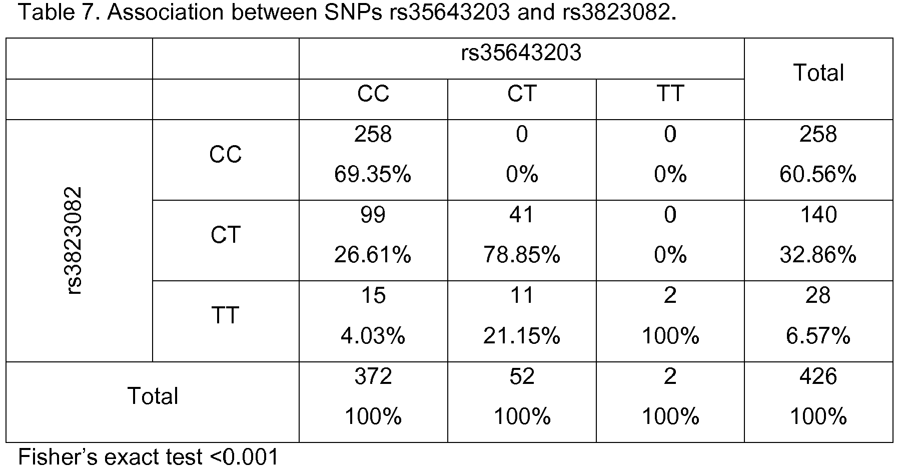

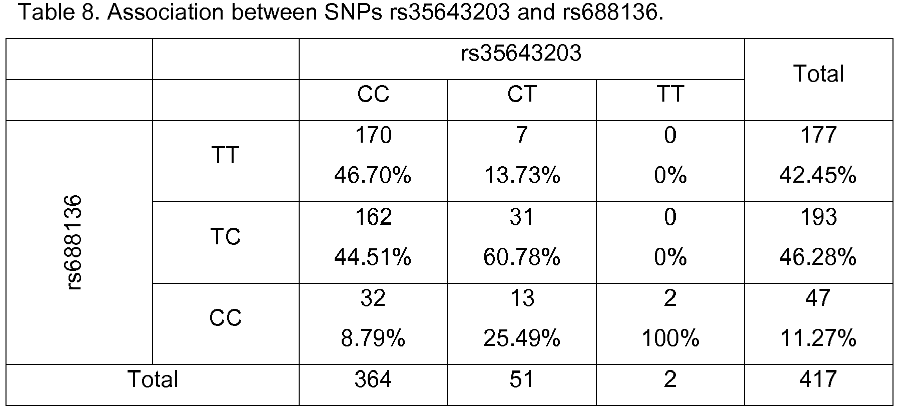

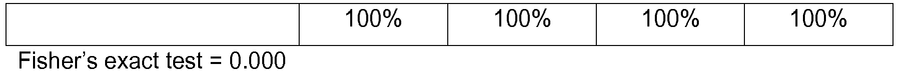

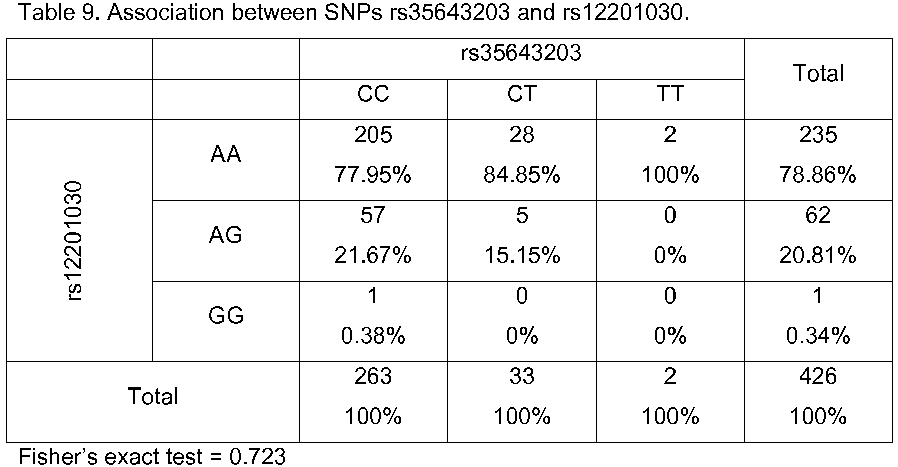

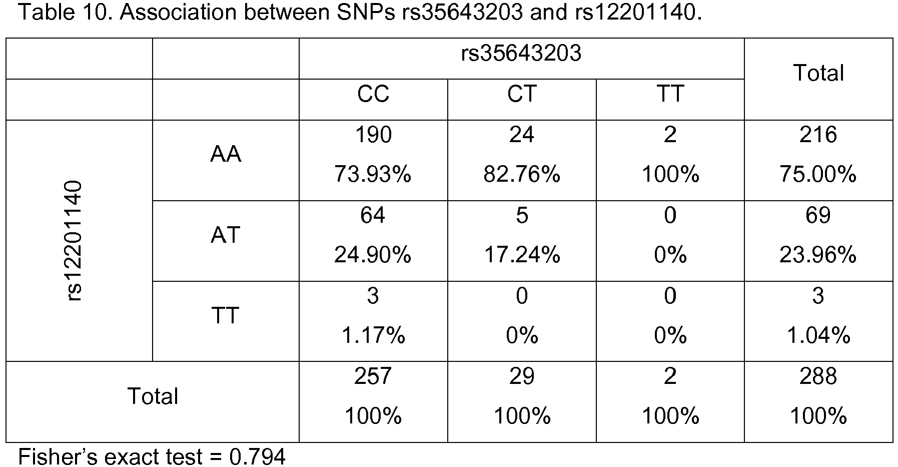

There is no relevant relationship between rs3823082, rs688136, rs12201030 and 12201140 either (data not shown).

### Example 2.1: Relationship between the genotype of the different variants and the VIP levels in the extended population with recent-onset arthritis

As shown in Figure 1, patients homozygous for the normal allele of rs35643203 (panel A, CC genotype) showed significantly higher serum VIP levels than patients carrying at least one T allele. Since rs35643203 is in linkage disequilibrium with rs71575932 and rs7755568, the serum VIP level pattern between genotypes is virtually identical (panels B and C, respectively). The differences in the levels of significance correspond to differences in the number of samples due to the lack of information about the genotype of some patients as a result of a technical error. As can be seen in the three cases, although the VIP levels are generally higher among the patients homozygous for the major allele, there are still patients with low VIP levels. This means that these genetic variants are not the only ones involved in regulating neuropeptide levels.

With respect to the SNP rs3823082, as seen in Figure 2, patients homozygous for the minor allele (T) are those having lower VIP levels. As has been mentioned when describing the preceding figure, also in the case of this SNP there are patients with low levels who have among them at least one copy of the normal (C) allele.

Figure 3 shows the VIP levels according to the genotypes of SNPs rs688136 (panel A) and rs12201140 (panel B). In the sequencing study, the minor allele of rs688136 was found more often among patients with high VIP, particularly the CC genotype. This trend is observed in the total population from which serum VIP levels are obtained, although it does not reach significant levels (Figure 3A). This situation seems to be related to interaction with rs12201140. In the case of this SNP, the minor allele (T) was only found in one of the patients with low VIP as a heterozygous genotype. In a more extended population, that trend of serum VIP levels being lower is observed in heterozygous AT, but that trend is missing in homozygous TT. The explanation is that these four patients who are homozygous TT of rs12201140, who should have lower levels than heterozygous AT, are all homozygous CC of rs688136 which is associated with higher levels. This suggests that the different variants that have been found interact with one another, so more complex statistical studies that are described below are required.

With respect to rs12201030, the available information suggests that it has no influence on serum VIP levels (data not shown).

The study of the interactions between the different genetic variants was started by analyzing if there was an additive effect between variants clearly associated with low VIP: having at least one minor allele of rs35643203, rs71575932 or rs7755568 (in linkage disequilibrium) and being homozygous for the minor allele of rs3823082. As shown in Figure 4, the more genetic variants associated with low VIP, the lower the VIP levels will be, increasing the statistical significance.

Figure 5 shows how being homozygous CC of rs688136 modulates the effect described in Figure 4 towards higher VIP levels.

In summary, at least 5 out of the 17 SNP-type genetic variants detected in Example 1) are associated with a differential distribution of VIP levels. Therefore they could be used as prognostic biomarkers instead of the measurement of VIP levels. The genotypes providing more information individually are rs35643203, rs71575932 and rs7755568 that may be interchanged since their distribution is virtually identical (Tables 4 to 6). In turn, rs3823082 provides additional information about a subpopulation of patients, homozygous for the minor allele, also contributing to the patients having lower serum VIP levels. The additive effect of these four SNPs is clearly seen in Figure 4. Finally, the CC genotype of rs688136 is associated with having higher serum VIP levels.

The interaction between these genetic variants is clearly seen in the multivariate analysis shown in Table 11 which has also included variables such as age and use of TNF blocking agents which were demonstrated in an earlier study to be associated significantly with an increase in VIP levels (Martínez C *et al.* 2014 PLOS One 1:e85248). In the model without interaction between rs35643203 (as the representative of the three SNPs that are in linkage disequilibrium: rs35643203, rs71575932 and rs7755568) and rs3823082, it is observed that in the case of the first SNP there are negative coefficients (indicating association with lower VIP levels) in a dose-dependent manner with respect to the presence of the minor allele T which reaches statistical significance in heterozygous cases (as a result of having a large sample size). In the case of rs3823082, the coefficients also are negative although statistical significance is not reached. When the model with interaction is carried out, an additive effect is observed between the two minor alleles of rs35643203 and rs3823082. In both models, the CC genotype of rs688136 has positive and highly significant coefficients.

**Table 11. Effect of different genetic variants in the VIP gene on VIP serum levels, adjusted for age and use of TNF blocking drug.**

| | Model without interaction* | | Model with interaction** | |
|---|---|---|---|---|
| | β Coef. ± s.e. | P | P Coef. ± s.e. | P |
| Age | 2.6 ± 1.1 | 0.016 | 2.5 ± 1.1 | 0.019 |
| Use of anti-TNF therapy | 136 ± 57 | 0.017 | 128 ± 57 | 0.026 |
| rs35643203/rs3823082 | | | | |
| CC/CC | | | Reference | - |
| CC/CT | | | -40 ± 46 | 0.388 |
| CC/TT | | | 128 ± 138 | 0.353 |
| CT/CC | | | No data | - |
| CT/CT | | | -117 ± 64 | 0.066 |
| CT/TT | | | -310 ± 98 | 0.002 |
| TT/CC | | | No data | - |
| TT/CT | | | No data | - |
| TT/TT | | | -363 ± 174 | 0.037 |
| rs35643203 | | | | |
| CC | Reference | - | | |
| CT | -131 ± 66 | 0.046 | | |
| TT | -239 ± 181 | 0.187 | | |
| rs3823082 | | | | |
| CC | Reference | - | | |
| CT | -25 ± 46 | 0.58 | | |
| TT | -75 ± 94 | 0.424 | | |
| Genotype rs688136 | | | | |
| TT | Reference | - | Reference | - |
| TC | 51 ± 34 | 0.133 | 44 ± 34 | 0.185 |
| CC | 211 ± 74 | 0.004 | 235 ± 74 | 0.002 |

| | | | | |
|---|---|---|---|---|
| Coef: coefficient; s.e.: standard error. p<0.05 is considered statistically significant. *The model without interaction studies the effect of the genotypes of rs35643203 and rs3823082 separately. **The model with interaction studies the effect of the different combinations of the genotypes of rs35643203 and rs3823082. For this reason, there are empty cells in the table. | | | | |

### Example 2.2: Study of genetic variants rs3823082, rs35643203 and rs71575932 as prognostic markers in patients with early arthritis

To analyze if the mentioned genetic variants or their combination were useful for predicting the prognosis of patients with early arthritis, three parameters relating to the severity of the disease were used:
- disease activity estimated by means of DAS28 composite indices (the current gold standard) and HUPI, a recently described index for disease evaluation (Castrejon et al. Arthritis Care Res 2013;65:518-25) that is less biased compared to DAS28.
- treatment requirement in the first two years of follow-up. Given that it is not ethically acceptable to study the natural history of the disease without intervention (DMD treatment) which would provide the ideal and irrefutable setting for prognostic factor study, it is considered acceptable to use treatment requirement variables as subrogated variables of disease activity or severity. In this sense, DMD treatment intensity was used, calculated as the sum of the number of days the patient has been taking the different DMDs they received in the first two years of follow-up, weighted by a coefficient according to the strength of the DMDs, based on the number of days that elapsed between the baseline visit and two-year follow-up visit (for more accurate definition, see Gonzalez-Alvaro *et al.* PLoS One 2011; 6(12):e29492)
- radiological progression in the first two years of follow-up measured according to the Van der Heijde-modified Sharp score (available in only 91 patients), known by the person skilled in the art.

As regards disease activity throughout the follow-up process, no differences were observed between the genotypes of the SNPs individually with any one of the two activity indices used (data not shown). When the patients were clustered according to the number of genetic variants associated with low serum VIP (taking into account the 4 SNPs described in Figure 4), an insignificant trend was observed, as there is a higher activity level in patients carrying the variants related with lower VIP levels (Figure 6), i.e., being homozygous for T of rs3823082 and having at least one copy of the minor allele of rs35643203, rs71575932 or rs7755568.

These differences were more pronounced in visits 2 and 3, although statistical significance was never reached (best case scenario in visit 3, p=0.113). Logically, throughout follow-up, treatment was intensified in those patients who were observed to have persistent disease activity, and for this reason virtually no differences were observed between the 3 groups of patients in the final visit.

For this reason, a multivariate analysis was conducted to study the effect of the genotypes of VIP genetic variants on disease activity progression, adjusted for other variables that affect disease activity (sex, age, treatment, presence of ACPA) which have already been described. As seen in Table 12, being older than 45 years of age is associated with higher values of the indices evaluating disease activity (DAS28 of C-reactive protein, [PCR] or HUPI) and being a woman is associated with having higher PCR DAS28 values. Logically, the use of different DMDs and anti-TNF therapy were associated with reduced disease activity. Adjusting for these variables, having 2 genetic variants associated with low serum VIP levels was significantly associated with having higher levels of disease activity in ACPA negative patients. In the case of rs688136, having a CC genotype showed an insignificant trend of having lower disease activity.

**Table 12. Multivariate analysis of the effect of VIP genetic variants on activity progression in patients with early arthritis.**

| | DAS28 | | HUPI | |
|---|---|---|---|---|
| | β Coef. ± s.e. | p | β Coef. ± s.e. | p |
| Sex | | | | |
| Male | Reference | - | Reference | - |
| Female | 0.32±0.15 | 0.037 | 0.48±0.34 | 0.162 |
| Age | | | | |
| <45 years | Reference | Reference | Reference | - |
| 45-65 years | 0.35±0.15 | 0.021 | 0.87±0.34 | 0.01 |
| >65 years | 0.26±0.17 | 0.129 | 0.64±0.38 | 0.94 |
| Methotrexate | -0.81±0.08 | <0.001 | -1.80±0.19 | <0.001 |
| Leflunomide | -0.66±0.11 | <0.001 | -1.54±0.26 | <0.001 |
| Anti-malarial drugs | -0.50±0.12 | <0.001 | -1.35± 0.29 | <0.001 |
| Sulfasalazine | -0.45±0.19 | 0.019 | -1.12±0.45 | 0.01 |
| Gold salts | -1.88±0.49 | <0.001 | -3.13±1.05 | 0.003 |
| Anti-TNF | -0.92±0.19 | <0.001 | -2.60±0.45 | <0.001 |
| ACPA/Low VIP genotypes* | | | | |
| Negative/0 | Reference | - | Reference | - |
| Negative/1 | 0.18±0.26 | 0.482 | 0.01±0.60 | 0.984 |
| Negative/2 | 0.94±0.50 | 0.058 | 2.02±1.13 | 0.075 |
| Positive/0 | 0.24±0.14 | 0.087 | 0.52±0.32 | 0.106 |
| Positive/1 | 0.11±0.25 | 0.656 | 0.42±0.57 | 0.462 |
| Positive/2 | 0.06±0.60 | 0.912 | 0.51±1.39 | 0.715 |
| rs688136 | | | | |
| TT | Reference | - | Reference | - |
| TC | 0.13±0.13 | 0.323 | 0.25±0.31 | 0.414 |
| CC | -0.24±0.22 | 0.291 | -0.71±0.52 | 0.172 |

| | | | | |
|---|---|---|---|---|
| *Label 0 corresponds to not being homozygous for T of rs3823082 and not having any minor allele of rs35643203, rs71575932 or rs7755568. Label 1 corresponds to being homozygous for T of rs3823082 or having at least one minor allele of rs35643203, rs71575932 or rs7755568. Label 2 corresponds to being homozygous for T of rs3823082 and having at least one minor allele of rs35643203, rs71575932 or rs7755568. | | | | |

The next variable to be analyzed was DMD treatment intensity throughout the two-year follow-up. This variable can be considered a subrogated variable of severity since, as mentioned in the preceding paragraph, those patients having poorer progression in follow-up are being treated more intensely.

Like with disease activity, the genotype of the isolated SNPs did not provide solid information (data not shown). Figure 7 shows the treatment requirements according to the combinations of the genotypes of rs3823082, rs35643203, rs71575932 and rs7755568 which are associated with having a low VIP. It is again observed that patients having the association of genotypes for a low VIP require a more intense treatment, in a more homogenous manner. In a simple analysis, these differences do not reach statistical significance (p=0.3). It is well established through earlier studies conducted by the rheumatology community that the presence of ACPAs (anti-citrullinated protein antibodies) or the female gender are factors of a poor prognosis. This knowledge leads to an intervention bias, i.e., it causes treating physicians to prescribe an early intensive treatment when any of the factors is present, and for this reason, in the analysis of the relationship between VIP genetic variants and treatment intensity, ACPA and the female gender act as modifying factors or factors of confusion.

For this reason, a multivariate analysis was conducted confirming that women, patients who complied with RA criteria, and almost significantly those who have ACPA, received higher intensity treatment (Table 13). Adjusting for these variables of confusion, it was verified that patients having genetic variants associated with having low VIP levels (i.e., being homozygous for T of rs3823082 and having at least one copy of the minor allele of rs35643203, rs71575932 or rs7755568) received a significantly more intense treatment (Table 13 and Figure 8).

**Table 13. Variables associated with the prescription of a more intense DMD treatment in patients with recent-onset arthritis.**

| | Coefficient | Standard Error | p |
|---|---|---|---|
| Sex | | | |
| Male | Reference | - | - |
| Female | 0.36 | 0.12 | 0.003 |
| Diagnosis | | | |
| Undifferentiated arthritis | Reference | - | <0.001 |
| Rheumatoid arthritis | 0.85 | 0.12 | |
| ACPA | | | |
| Negative | Reference | - | 0.072 |
| Positive | 0.20 | 0.11 | |
| Genetic variants associated with low serum VIP | | | |
| Zero | Reference | - | - |
| One | -0.14 | 0.15 | -0.344 |
| Two | 0.77 | 0.35 | 0.028 |
| rs688136 | | | |
| TT | Reference | - | - |
| TC | 0.01 | 0.11 | 0.929 |
| CC | -0.14 | 0.19 | 0.441 |

The condition of each variable considered to be the reference in the model is the condition that is being compared to the other conditions of said variable. For example, adjusted for the effect of the rest of the variables, being a woman is associated with receiving treatment 0.36 times more than men do.

Figure 8 allows visualizing how variables such as sex, diagnosis and ACPA introduce a bias or cause confusion/modification with respect to the effect of the variable of interest (low or normal VIP levels) on the dependent (or outcome) variable, in this case DMD treatment intensity. It can therefore be seen that the higher treatment intensity associated with having the genetic variants which determine having a low VIP (being homozygous for T of rs3823082 and having at least one copy of the minor allele of rs35643203, rs71575932 or rs7755568) is observed better in the case of male patients, with undifferentiated arthritis or negative ACPA which will not lead the rheumatologist to a more intensive treatment from the onset of the condition. In patients who comply with the RA criteria, those who are women or those who have positive ACPAs, treating physicians tend to always provide a more intense treatment, although a certain trend for more treatment is also observed in patients having more genetic variants associated with low VIP (label 2).

As regards the use of combined therapy, those patients who had more alleles associated with a low serum VIP received this type of treatment with a higher frequency (62.5%) than those patients who did not have any allele associated with a low VIP (46.03%). The differences did not reach statistical significance, probably because in order to achieve faster disease control, the use of combined therapy is a very common practice among rheumatologists at HU La Princesa.

Finally, a study was conducted in a more limited population on how the presence of genetic variants affects joint destruction measured with van der Heijde-modified Sharp score (Van der Heide DM et al. J Rheumatol 1995;22(9):1792-6). As seen in Figure 9, patients having no genetic variants associated with a low serum VIP had lower overall radiological progression than patients having at least one genetic variant. These differences almost reached statistical significance (p=0.062). A multivariate analysis was again conducted in which variables known to affect radiological progression such as the presence of the shared epitope, being a smoker or the sex, were included.

**Table 14. Variables associated with the progression of joint destruction in patients with recent-onset arthritis estimated in hand and leg x-rays by means of the van der Heijde-modified Sharp score.**

| | Coefficient | Standard error | P |
|---|---|---|---|
| Sex | | | |
| Male | Reference | - | - |
| Female | -9 | 3.9 | 0.020 |
| Smoking habit | | | |
| Non smoker | Reference | - | - |
| Ex-smoker | -8.7 | 6.7 | 0.196 |
| Smoker | 10.5 | 4.2 | 0.013 |
| Number of copies of EC | | | |
| Zero | Reference | - | - |
| One | 4.2 | 3.5 | 0.231 |
| Two | 13.8 | 5.7 | 0.016 |
| Genetic variants associated with low serum VIP | | | |
| No | Reference | - | |
| Yes | 13.1 | 4.9 | 0.007 |
| rs688136 | | | |
| TT | | | |
| TC | N.I. | N.I. | - |
| CC | | | |

| | | | |
|---|---|---|---|
| S.E.: shared epitope (refers to the common amino acid sequence encoding those alleles of HLA-DRB1 which are associated with a higher risk of developing rheumatoid arthritis). N.I. not included so as to not improve the model. | | | |

### Example 3: Relationship between the genotype of the different variants and VIP levels in a population with recent-onset spondyloarthritis

To determine if the findings described above can be extended to other immune mechanism-mediated diseases, a study was conducted on 54 patients from this practice with early spondyloarthritis in whom serum VIP levels as well as those genetic variants which had an influence on VIP levels (rs3823082, rs71575932, rs35643203, rs688136) in the population with early arthritis were determined. In this study, rs7755568 was not genotyped because it is in linkage disequilibrium with rs71575932 and rs35643203.

Clinical data of patients is described in Table 15.

**Table 15. Baseline characteristics of patients from the practice with early spondyloarthritis according to their final diagnosis.**

| | Spondyloarthritis (n=36) | Chronic lower back pain (n=18) | Total (n=54) | p |
|---|---|---|---|---|
| Age (years) | 36..1 ± 10.7 | 40.1 ± 10.1 | 37.5 ± 10.6 | N.S. |
| Female (%) | 24 (66.7) | 9 (50.0) | 33 (61.1) | N.S. |
| Family history of SpA (%) | 14 (38.9) | 1 (5.6) | 15 (27.8) | 0.042 |
| HLA-B27 positive (%) | 21 (58.3) | 3 (16.7) | 24 (44.4) | 0.012 |
| Enthesitis | 15 (41.7) | 4 (22.2) | 19 (35.2) | N.S. |
| Peripheral arthritis | 11 (30.6) | 1 (5.6) | 12 (22.2) | N.S. |
| Uveitis | 3 (8.3) | 1 (5.6) | 4 (7.4) | N.S. |
| Psoriasis | 4 (11.1) | 1 (5.6) | 5 (9.3) | N.S. |
| IBD** | 2 (5.6) | 0 (0.0) | 2 (3.7) | N.S. |
| Sacroiliitis MR ** | 21 (58.8) | 2 (11.1) | 23 (42.6) | 0.004 |
| Sacroiliitis X-ray** | 12 (33.3) | 0 (0.0) | 12 (22.2) | N.S. |
| BASDAI * | 41.2 ± 22.9 | 35.6 ± 22.4 | 39.4 ± 22.7 | N.S. |
| BASFI*** | 29.0 ± 34.5 | 24.0 ± 33.0 | 25.5 ± 34.0 | N.S. |
| PCR (mg/dl) * | 0.3 ± 1.0 | 0.1 ± 0.2 | 0.2 ± 0.7 | N.S. |
| ESR (mm/h)* | 16.5 ± 19.5 | 11.5 ± 13.0 | 14.0 ± 17.0 | N.S. |
| Hb (g/dl)* | 13.2 ± 2.5 | 14.5 ± 1.3 | 13.7 ± 2.2 | 0.025 |
| VIP (pg/ml) * | 250.4 ±75.2 | 271.5±111.9 | 250.7±91.7 | N.S. |

| | | | | |
|---|---|---|---|---|
| IBD: Inflammatory bowel disease; MR: Magnetic resonance; BASDAI: Bath Ankylosing Spondylitis Disease Activity Index; BASFI: Bath Ankylosing Spondylitis Functional Index; PCR: C-reactive protein; ESR: erythrocyte sedimentation rate; Hb: hemoglobin; VIP: vasoactive intestinal peptide; N.S.: not significant. | | | | |

In this new population, it was verified that as occurred in the population with early arthritis, rs71575932 and rs35643203 are in linkage disequilibrium since their genotype is virtually identical in all patients (Table 16).

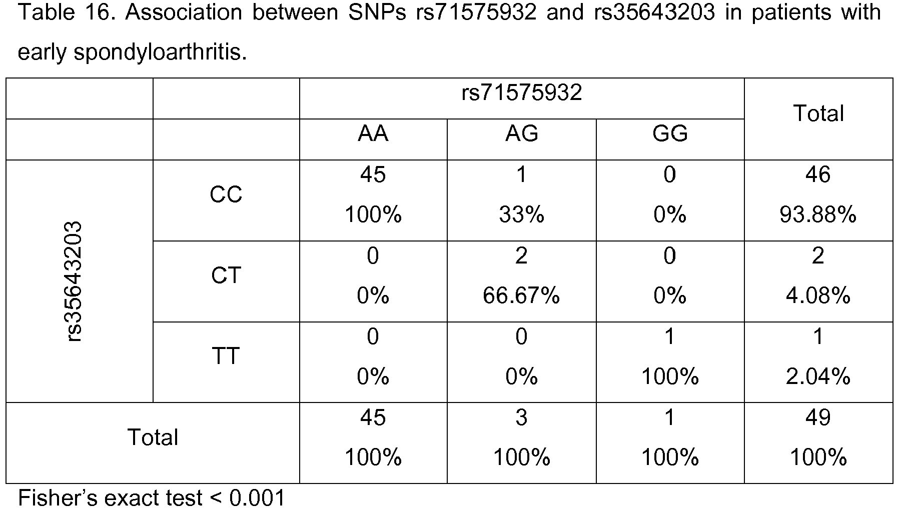

Therefore, it can be concluded that both polymorphisms can be used interchangeably. This situation was different with rs3823082 and rs688136, as seen in Tables 17 and 18, respectively. As occurred with patients with early arthritis, rs3823082 had a distribution similar to rs71575932 and rs35643203, but with a relevant subpopulation having genotypes that do not coincide (Table 17).

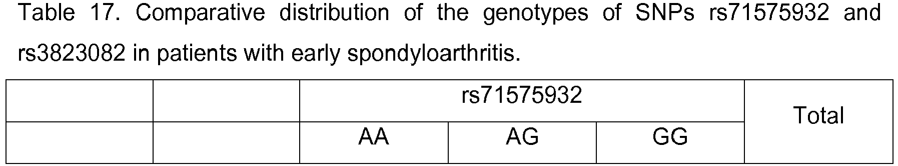

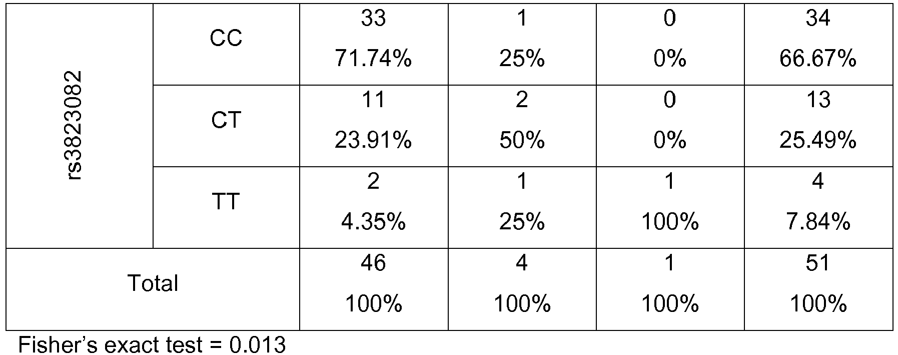

The differences in the genotypes of rs71575932 or rs35643203 and rs688136 were even more different (Table 18), as occurred in Example 1 in patients with early arthritis.

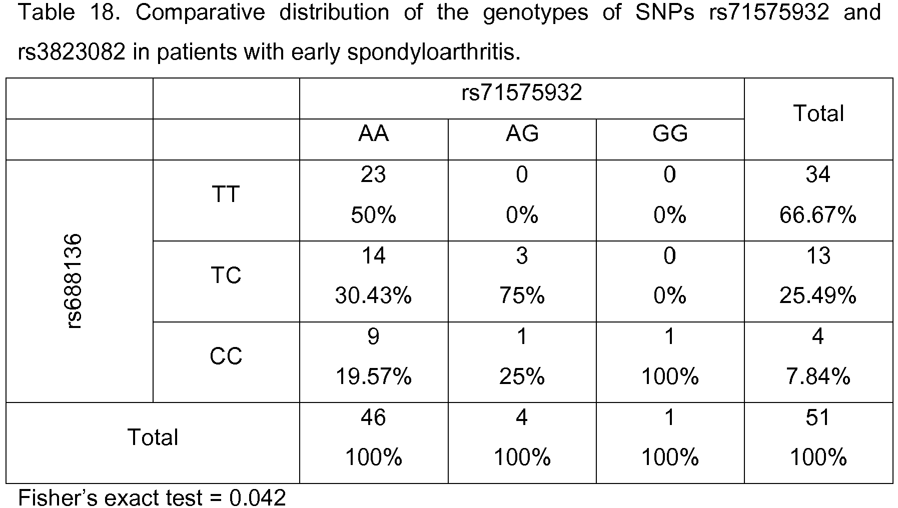

To determine how the studied VIP genetic variants influence serum levels of this neuropeptide in patients with early spondyloarthritis, a multivariate analysis was carried out, similarly adjusted to that shown in Example 2.1 (Table 11) for patients with early arthritis. The results obtained are similar in both cases, with the exception that the values of significance p are lower in spondyloarthritis patient analysis since the sample size was much smaller. Table 19 shows that having a minor allele of the SNPs associated with a low VIP in early arthritis (rs3823082, rs71575932, rs35643203) was also associated with lower serum VIP levels in early spondyloarthritis. In contrast, being a carrier of at least one minor allele C of rs688136 was associated with higher serum VIP levels (Table 19). The only difference is that in the case of spondyloarthritis patients, age was not shown to be associated with the serum VIP levels, probably because spondyloarthritis patients tend to develop the disease at an overall younger age than rheumatoid arthritis patients, in whom there is a greater variability and the age spectrum is broader so as to detect the small association described in Table 11.

Figure 11 graphically shows how being a carrier of the minor allele of rs688136, in the absence of a minor allele of rs3823082, rs71575932 or rs35643203, is associated with the presence of higher VIP levels (white box on the left side of the drawing). In contrast, having a minor allele of rs3823082, rs71575932 or rs35643203, in the absence of the minor allele of rs688136, causes lower VIP levels (dark gray box on the right side of the drawing). How they interact with one another between is also observed, such that the presence of the minor allele of rs688136 compensates for the effect of having a minor allele of rs3823082, rs71575932 or rs35643203 and the levels in this case are intermediate and similar to when there was no minor allele of these four variants (central boxes in the drawing).

**Table 19. Effect of different genetic variants in the VIP gene on serum VIP levels (normalized by means of log transformation), adjusted for the use of TNF blocking drug.**

| | β Coefficient | Standard Error | p |
|---|---|---|---|
| Use of anti-TNF therapy | 0.13 | 0.07 | 0.045 |
| At least one minor allele of rs3823082, rs71575932 or rs35643203 | | | |
| Absent | Reference | - | - |
| Present | -0.12 | 0.1 | 0.211 |
| Genotype rs688136 | | | |
| TT | Reference | - | - |
| TC or CC | 0.17 | 0.1 | 0.072 |

| | | | |
|---|---|---|---|
| Coef: coefficient; s.e.: standard error. p<0.05 is considered statistically significant | | | |

### Example 4: Relationship between the genotype of the different variants and VIP levels in a population with systemic lupus erythematosus

To determine if the findings described above can be extended to systemic lupus erythematosus, a study was conducted with 78 sera from patients with said disease. As a conclusion, data similar to the data referring to rheumatoid arthritis and spondyloarthritis is observed.

## Claims

1. A method for obtaining data useful for the prognosis of an autoimmune disease which comprises detecting genetic polymorphisms rs35643203, rs71575932 and/or rs7755568 of the "vasoactive intestinal peptide" (*VIP*) gene in a biological sample isolated from a subject suffering from an autoimmune disease.

2. The method according to claim 1, which further comprises detecting genetic polymorphism rs3823082 and/or rs688136 of the *VIP* gene.

3. The method according to claim 2, which further comprises detecting at least one of the genetic polymorphisms of the *VIP* gene selected from the list comprising: rs12213214, rs60946248, rs140023105, rs7764067, rs12201030, rs74760293, rs149081483 and rs12201140, or any of the combinations thereof.

4. The method according to any of claims 1 to 3, where the autoimmune disease is selected from the list comprising: autoimmune arthritis, spondyloarthritis, systemic lupus erythematosus, psoriasis and inflammatory bowel disease.

5. The method according to claim 4, where the autoimmune arthritis is rheumatoid arthritis.

6. The method according to any of claims 1 to 5, where the biological sample is blood, serum, plasma, saliva, urine, synovial fluid or lymph.

7. The method according to any of claims 1 to 6, where the subject is a human.

8. An *in vitro* method for the prognosis of an autoimmune disease in a subject suffering from an autoimmune disease, which comprises:
a. detecting genetic polymorphisms rs35643203, rs71575932 and/or rs7755568 of the *VIP* gene in a biological sample; and
b. associating the T allele of genetic polymorphism rs35643203, associating the G allele of genetic polymorphism rs71575932 and/or associating the A allele of polymorphism rs7755568, with a poor prognosis.

9. The method according to claim 8, which further comprises in step (a) detecting polymorphism rs3823082 and/or rs688136 of the *VIP* gene and in step (b) associating the TT genotype of polymorphism rs3823082 with a poor prognosis and/or associating the CC genotype of polymorphism rs688136 with a good prognosis.

10. The method according to claim 9, which further comprises detecting in step (a) at least one of the genetic polymorphisms of the *VIP* gene selected from the list comprising rs12213214, rs60946248, rs140023105, rs7764067, rs12201030, rs74760293, rs149081483 and rs12201140, or any of the combinations thereof, and associating in step (b) the A allele of polymorphism rs12213214, the T allele of polymorphism rs60946248, the G allele of polymorphism rs140023105, the T allele of polymorphism rs7764067, the G allele of polymorphism rs12201030, the C allele of polymorphism rs74760293, the G allele of polymorphism rs149081483 and the T allele of polymorphism rs12201140, or any of the combinations thereof, with a poor prognosis.

11. The method according to any of claims 8 to 10, where furthermore anti-citrullinated protein antibodies are detected in a step (c).

12. The method according to any of claims 8 to 11, where the autoimmune disease is selected from the list comprising: autoimmune arthritis, spondyloarthritis, systemic lupus erythematosus, psoriasis and inflammatory bowel disease.

13. The method according to claim 12, where the autoimmune arthritis is rheumatoid arthritis.

14. The method according to any of claims 8 to 13, where the biological sample is blood, plasma, serum, saliva, urine, synovial fluid or lymph.

15. The method according to any of claims 8 to 14 where the subject is a human.

16. An *in vitro* method for designing a personalized treatment for a subject suffering from an autoimmune disease which comprises detecting genetic polymorphisms rs35643203, rs71575932 and/or rs7755568 of the *VIP* gene in the biological sample, wherein the presence of the T allele of genetic polymorphism rs35643203, the presence of the G allele of genetic polymorphism rs71575932 and/or the presence of the A allele of polymorphism rs7755568 is indicative that the treatment to be administered is a combined therapy of synthetic and biological disease-modifying drugs (DMDs).

17. The method according to claim 16, which further comprises detecting polymorphism rs3823082 and/or rs688136 of the *VIP* gene and where the presence of the TT genotype of polymorphism rs3823082 is indicative that the treatment to be administered is said combined therapy and/or the presence of the CC genotype of polymorphism rs688136 is indicative that the treatment to be administered is a monotherapy of non-biological DMD.

18. The method according to claim 17, which further comprises detecting the polymorphism of the *VIP* gene selected from the list comprising rs12213214, rs60946248, rs140023105, rs7764067, rs12201030, rs74760293, rs149081483 and rs12201140, or any of the combinations thereof; and where the presence of the A allele of polymorphism rs12213214, the T allele of polymorphism rs60946248, the G allele of polymorphism rs140023105, the T allele of polymorphism rs7764067, the A allele of polymorphism rs7755568, the C allele of polymorphism rs74760293, the G allele of polymorphism rs149081483 and the T allele of polymorphism rs12201140, or any of the combinations thereof, is indicative that the treatment to be administered is said combined therapy and/or the presence of the G allele of polymorphism rs12201030 is indicative that the treatment to be administered is a monotherapy of non-biological DMD.

19. The method according to any of claims 16 to 18, where the autoimmune disease is selected from the list comprising: autoimmune arthritis, spondyloarthritis, systemic lupus erythematosus, psoriasis and inflammatory bowel disease.

20. The method according to claim 19, where the autoimmune arthritis is rheumatoid arthritis.

21. The method according to any of claims 16 to 20, where the biological sample is blood, plasma, serum, saliva, urine, synovial fluid or lymph.

22. The method according to any of claims 16 to 21, where the biological DMD is a tumor necrosis factor blocking agent.

23. The method according to any of claims 16 to 22, where the subject is a human.

24. Use of genetic polymorphisms rs35643203, rs71575932 and/or rs7755568 of the *VIP* gene as a prognostic marker of an autoimmune disease in a subject suffering from an autoimmune disease.

25. Use according to claim 24, further comprising the use of genetic polymorphism rs3823082 and/or rs688136 of the *VIP* gene.

26. Use according to claim 25, further comprising the use of genetic polymorphisms of the *VIP* gene selected from the list comprising: rs12213214, rs60946248, rs140023105, rs7764067, rs12201030, rs74760293, rs149081483 and rs12201140, or any of the combinations thereof.

27. Use according to any of claims 24 to 26, where the autoimmune disease is selected from the list comprising: autoimmune arthritis, spondyloarthritis, systemic lupus erythematosus, psoriasis and inflammatory bowel disease.

28. Use according to claim 27, where the autoimmune arthritis is rheumatoid arthritis.

29. Use according to any of claims 24 to 28, where the subject is a human.

30. A kit comprising primers and/or probes for detecting genetic polymorphisms rs35643203, rs71575932 and/or rs7755568 of the *VIP* gene.

31. The kit according to claim 30, further comprising primers and/or probes for detecting genetic polymorphism rs3823082 and/or rs688136 of the *VIP* gene.

32. The kit according to claim 31, further comprising primers and/or probes detecting genetic polymorphisms rs12213214, rs60946248, rs140023105, rs7764067, rs12201030, rs74760293, rs149081483 and rs12201140 of the VIP gene, or any of the combinations thereof.

33. The kit according to any of claims 30 to 32, further comprising antibodies, probes or primers specific for detecting anti-citrullinated protein antibodies or citrullinated peptides or citrullinated proteins.

34. Use of the kit according to any of claims 30 to 33 for the prognosis of an autoimmune disease in a subject suffering from said disease.

35. Use according to claim 34, where the autoimmune disease is selected from the list comprising: autoimmune arthritis, spondyloarthritis, systemic lupus erythematosus, psoriasis and inflammatory bowel disease.

36. Use according to claim 35, where the autoimmune arthritis is rheumatoid arthritis.

37. Use according to any of claims 34 to 36, where the subject is a human.
